# EUROPÄISCHE PATENTANMELDUNG

(11) **EP 2 255 774 A1**
(43) Veröffentlichungstag der Anmeldung: **01.12.2010**
(21) Anmeldenummer: 09405086.1
(22) Anmeldetag: 25.05.2009
(51) Int. Cl.: A61J 7/00

(54) **Medikamentenrichtstation**

(71) Anmelder: Wiegand AG, 8180 Bülach (CH)
(72) Erfinder: Wiegand, Markus, 8182 Hochfelden (CH); Hils, Karin, 8426 Lufingen (CH)
(74) Vertreter: Rüfenacht, Philipp Michael

(57) **Zusammenfassung**

Eine Vorrichtung zur Unterstützung eines manuellen Richtens von Medikamenten umfasst eine Halterung (3.3) für einen Medikamentenabgabebehälter (4), welcher wahlweise in ein oder mehrere Fächer (4.1, 4.2, 4.3, 4.4) unterteilbar ist, ein elektronisch ansteuerbares Fachzeigemittel (3.1, 3.11) zum visuellen Hinweisen auf das zu befüllenden Fachs (4.1, 4.2, 4.3, 4.4) des Medikamentenabgabebehälters (4), und ein Steuermodul (2.51) zur Steuerung des Fachzeigemittels (3.1, 3.11) aufgrund von vorgegebenen Verschreibungsdaten. Ein System zum Richten und Verteilen von Medikamenten umfasst eine Medikamentenrichtstation (1) mit einem Schrank (2) zur Lagerung eines Medikamentenvorrats, die erwähnte Vorrichtung zur Unterstützung eines manuellen Richtens von Medikamenten, eine elektronischen Schaltung (2.5) mit einem Steuermodul (2.51), einem Codeerfassungsmodul (2.52) und/oder einem Einfütlkontrollmodul (2.53) sowie ein Eingabegerät zur manuellen Steuerung der elektronischen Schaltung (2.5). Als Grundlage dienen die von einem Arzt verordneten Medikamente.

## Beschreibung

### Technisches Gebiet

Die Erfindung betrifft eine Vorrichtung zur Unterstützung eines manuellen Richtens von Medikamenten, ein System zum Richten und Verteilen von Medikamenten und ein Verfahren zum Betreiben sowie ein Computerprogrammprodukt zur Steuerung eines solchen Systems.

### Stand der Technik

In Spitälern, Krankenhäusern, Pflegeheimen oder Altersheimen besteht ein hoher Bedarf nach einer exakten Abgabe von Medikamenten.

Der Medikationsprozess respektive die Abgabe von Medikamenten kann in die Arbeitsschritte "Verschreiben" (Prescribing), "Übertragen" (Transcribing/Documenting), "Richten" (Dispensing), "Ausüben" (Administering) und "Überwachen" (Monitoring) unterteilt werden. Das Verschreiben eines Medikaments erfolgt durch den behandelnden Arzt, nachdem dieser den Patienten untersucht hat. Die verschriebenen Medikamente werden danach in eine Patientenakte übertragen. Aufgrund der Patientenakte werden Medikamente gerichtet und in Medikamentenabgabebehälter respektive Medikamenten-Dispenser bereitgestellt. Das Richten von Medikamenten wird manchmal auch als Rüsten oder als Vorbereiten der Medikamente bezeichnet.

Da Medikamente regelmässig eingenommen werden müssen, oft sogar mehrmals täglich wie beispielsweise am Morgen, am Mittag und am Abend, ist ein Medikamenten-Dispenser oft eingerichtet, um Medikamente eines Patienten für einen ganzen Tag aufzunehmen und bereitzustellen. Die Medikamente sind im Medikamenten-Dispenser beispielsweise in entsprechend beschrifteten und verschlossenen Fächern angeordnet, d.h. alle Medikamente, welche ein Patient beispielsweise am Morgen eines bestimmten Tages einnehmen muss, sind in einem entsprechend beschrifteten und verschlossenen Fach angeordnet. Jedes der Fächer entspricht einer bestimmten Abgabezeit oder hat eine spezielle Funktion wie beispielsweise zur Aufbewahrung von Reservemedikamenten oder Betäubungsmitteln. Beim Ausüben der Verabreichung von Medikamenten öffnet der Patient oder das Pflegepersonal das entsprechende Fach und es erfolgt eine Verabreichung der Medikamente. Die Wirkung der Medikamente wird dabei vom Pflegepersonal überwacht, d.h. es wird die Wirksamkeit überprüft oder es wird das Eintreten respektive Ausbleiben von Nebenwirkungen kontrolliert. Neben Medikamenten können mit einem Medikamenten-Dispenser auch andere Utensilien wie z.B. Spritzen, Verbandpflaster, Desinfektionsmittel, Nadeln etc. gerichtet und verteilt werden.

Aus der DE 26 17 540 (Wiegand) ist ein Tablett zur Aufbewahrung und Verteilung von Medikamenten bekannt. Das Tablett hat parallele Nuten zur Aufnahme von verschlossenen Medikamentenbehältern, die in getrennten Fächern die Ausgabeeinheiten für einen Patienten für einen Zeitabschnitt enthalten und eine mit Aufschrift versehbare Fläche aufweisen.

Aus der DE 25 36 342 (Wiegand) ist eine Vorrichtung zur Abgabe von Medikamenten bekannt. Medikamentenbehälter sind auf einem Tablett angeordnet. Der Medikamentenbehälter weist getrennte Fächer auf, eine parallel in Längsrichtung der Fächer verlaufende Schriftplatte und eine durchsichtige, dicht abschliessende, gleitbar angeordnete Abdeckung.

Das Richten oder Rüsten von Medikamenten kann im Voraus für einen oder mehrere Tage erfolgen. Fehler beim Richten entstehen durch Unkonzentriertheiten und können vermieden werden, indem diese Arbeit in einer dafür geeigneten Umgebung und in Ruhe ausgeführt wird. In Krankenhäusern wird oft in der Nachtschicht gerüstet, da dies eine besonders ruhige Periode im Tagesablauf ist. Das Richten von Arzneimitteln in einem grösseren Sinn-Zusammenhang, d.h. z.B. gleich für mehrere aufeinanderfolgende Tage, vermindert die Fehlerquote zusätzlich und erhöht die Effizienz. Es lohnt sich die Verteilung und Abgabe von Medikamenten optimal vorzubereiten, sodass trotz aller Hektik und Emotionalität im Umgang mit dem Patienten Fehler minimiert werden können. Deshalb ist ein Rüsten von Medikamenten direkt beim Patientenbett nicht sinnvoll.

Die verabreichende Pflegeperson überwacht die Verabreichung der Arzneimittel an den Patienten. Vordosierte, identifizierbare Arzneimittel und eine mitgeführte Pflegedokumentation sind ihre Hilfsmittel. Die Vordosierungen müssen sicher verpackt sein, so dass auch nach "kleinen Unfällen" wie etwa dem versehentlichen Herunterfallen der vordosierten Arzneimittel keine Unsicherheiten oder Verwechslungen von Medikamenten entstehen. Da die Medikamente meist von einer unterschiedlichen Pflegeperson gerüstet respektive abgegeben werden, erfolgt automatisch eine Kontrolle der Medikamente nach dem Vier-Augen-Prinzip. Die verabreichende Pflegeperson steht im direkten Kontakt zum Patienten und ist deshalb auch verantwortlich, den Verlauf der Therapie zu verfolgen und zu dokumentieren. Sehr gute Kenntnisse der Medikamente sind deshalb wichtig. Die Pflegeperson sollte die Wirkungen und Nebenwirkungen der Medikamente sehr genau kennen und möglichst auch über mögliche Interaktionen informiert sein.

Eine gute Organisationshilfe bei der medikamentösen Therapie sind die "WIEGAND MediDispenser". Dies sind hygienische und effiziente Hilfsmittel für die Unterstützung einer sicheren Vorbereitung, Verteilung und Abgabe von Arzneimitteln (und kleinen Hilfsmitteln wie Spritzen, Messbechern, etc.). Einmal gerüstet sind sie für die temporäre Lagerung der teilweise offenen Medikamente gasdicht und spritzwasserfest verschlossen (Untersuchung in einem Spital in England). Sie lassen sich bei der Verabreichung nur in einer Richtung öffnen, sodass ein zeitlich korrekter Ablauf bei der Medikamentenabgabe eingehalten wird. Sie sind günstig und können deshalb aus hygienischen Gründen (spezielle Kontaminationsgefahr) auch nur für eine einzige medikamentöse Therapie oder einen einzigen Patientenfall eingesetzt werden. "WIEGAND MediDispenser" gibt es in verschiedenen Grössen, so z.B. mit vier festen Fächern. Normalerweise werden die vier Fächer entsprechend den Abgabezeiten "Morgen", "Mittag" und "Abend" befüllt. Das letzte Fach wird für die Reserven oder für die Nachtabgabe benützt. Einige Pflegeheime verwenden auch 2 "MediDispenser": den einen für die Tagschicht und den anderen für die Nachtschicht. In Altersheimen werden die Dispenser oft für zwei oder für 7 Tage gerichtet. Die flexiblen, unterteilbaren "WIEGAND MediDispenser" lassen sich mit steckbaren Unterteilungen konfigurieren. Mit verschiedenen Fachgrössen und mit bis zu 12 Fächern können die unterteilbaren "MediDispenser" individuell an die Therapieformen und Arzneimittelgrössen angepasst werden. Die grössten konfigurierbaren "MediDispenser" eignen sich für ganzheitliche Rüst-und Verteilsysteme. Neben den Arzneimitteln wie Tabletten, Suppositorien, Sachets, Ampullen etc. lassen sich auch Hilfsmittel wie Becher, Einwegspritzen, Spritzen, Nadeln etc. geeignet richten, lagern und verteilen.

Es sind sogenannte Unit-Dose Systeme zur Medikamentenabgabe bekannt. Kommissioniermaschinen verpacken (verblistern) in einer zentralen Apotheke für jeden Patienten jedes Medikament einzeln und beschriften es mit für die Abgabe wichtigen Informationen (auch Barcode). Dann erfolgt die Auslieferung an die Stationen. Bei der Abgabe der Medikamente wird vom Pflegepersonal der Barcode vom Patientenarmband mit dem Barcode des einzeln verpackten Medikament verglichen und so sichergestellt, dass der richtige Patient das richtige Arzneimittel erhält. Grundsätzlich werden Blisterstreifen-, Blisterkarten- und Blistertütchensysteme unterschieden. Die Blisterstreifen- und Blisterkartensysteme eignen sich zum Verpacken von Tabletten und ähnlichen generischen Formen. Vertreter der Blisterstreifensysteme sind Yuyama (Baxter, Automed), JVM (HD MEDI) und Tosho. Vertreter der Blisterkartensysteme sind MTS, Manrex und Venalink. Ein Nachteil dieser Systeme ist, dass sie nur einfache und kleine galenische Formen - wie Tabletten - verpacken können und sie deshalb galenische Formen wie Spritzen, Salben, therapeutische Pflaster und Ampullen, die in Krankenhäusern und Kliniken zur Anwendung kommen, nicht abdecken können. Das System PillPick (Swisslog) verpackt nahezu alle Arzneimittelformen in spezielle Tütchen. Ein Nachteil der Swisslog Lösung ist ihre Komplexität, die grosse Investitions- und Unterhaltsaufwände erzwingt.

Der Hauptvorteil der Unit-Dose Systeme liegt darin, dass eine elektronische Verordnung erzwungen wird, die Arzneimittel automatisch für die Abgabe an den Patienten verpackt werden und somit Fehler in der Medikation erheblich vermindert werden. Nachteile der Unit-Dose Systeme sind, dass alle Arzneimittel umgepackt werden müssen und ein grosser Verpackungsaufwand entsteht, dass die Systeme sehr hohe Beschaffungskosten haben und deshalb nur zentralisiert gerichtet werden kann, dass durch die Zentralisierung ein grosser Transportaufwand und damit verbundene Zeitverluste zwischen Verschreibung und Medikamentenabgabe beim Patienten entstehen. Weitere Nachteile liegen darin, dass die Versorgungssicherheit der Patienten mit Arzneimitteln von einer zentralen Stelle abhängt, die nie ausfallen darf und die deshalb einen grossen Wartungsaufwand erzeugt. Ein weiterer Nachteil dieser Systeme liegt darin, dass die Prozesse der Arzneimittelverteilung stark verändert werden müssen, die Verantwortlichkeit vom Pflegepersonal in die Apotheke verlegt wird und deshalb die für die Patientensicherheit wichtige Überwachungsfunktion durch die Pflegefachleute geringer gewichtet wird.

Ein alternatives Verfahren zur Medikamenten Abgabe mit einem "WIEGAND MediDispenser" basiert auf dem sogenannten "amerikanischen" Medikamenten-Verteil-System, dem sogenannten BIN-System, bei dem auf ein vorbereitendes Richten der Medikation verzichtet wird. Die Arzneimittel werden in den Stationen für jeden Patienten separiert in kleinen Schubladen (BIN) aufbewahrt. Amerikanische Medikamentenwagen enthalten 12 bis 16 solcher BIN, die gemeinsam verschlossen werden können. Die Medikamente werden dezentral direkt vor der Abgabe beim Patienten gerichtet und abgegeben. Das heisst entweder im Stationszimmer oder gleich neben dem Patientenbett werden anhand der Patientendokumentation die zu diesem Zeitpunkt verordneten Arzneimittel dem BIN entnommen und gleich verabreicht. Es ist ein Nachteil, dass das 4-Augen-Prinzip fehlt. Die Konzentriertheit und Ruhe bei der Auswahl und Vorbereitung der Medikamente fehlen ebenfalls.

Das dezentrale manuelle Richten von Medikamenten kann in den Stationen elektronisch unterstützt werden. Dabei werden elektronisch zugriffsgeschützte Schubladensysteme verwendet, in die Arzneimittel abgefüllt werden. Aufgrund einer elektronischen Verordnung werden beim Richten jeweils nur freigegebene Arzneimittel zugreifbar gemacht. Nach der Anmeldung der Pflegeperson am System und der Identifikation des Patienten werden die Verordnungen angezeigt und der Reihe nach abgearbeitet, wobei die jeweils verordneten Arzneimittel durch das Öffnen einer Schublade freigegeben werden. Vertreter dieser elektronischen Schubladensysteme sind Pyxis, Omnicell und Vanas. Die Systeme ermöglichen eine elektronisch unterstützte Lagerführung, welche dem Apotheker eine bessere Übersicht über die Stationslager ermöglicht. Da nur ein bestimmtes Arzneimittel jeweils zugänglich ist, kann der Arzneimittelverlust durch eine unbeabsichtigte oder unbefugte Entnahme verringert werden. Beim Richten entstehen weniger Fehler, da das Richten elektronisch unterstützt ist. Der Hauptvorteil dieser Systeme liegt darin, dass eine elektronische Verordnung erzwungen wird und somit Fehler in der Medikation erheblich vermindert werden. Nachteile dieser Systeme liegen im hohen Platzbedarf und den hohen Investitionskosten. Ein weiterer Nachteil liegt darin, dass diese Systeme zwar die Medikamentenentnahme aus dem Stationslager unterstützen, nicht aber das patientenbezogene Richten (d.h. das Zusammenstellen der Medikamente eines Patienten) und Verteilen durch das Personal.

In einem unter dem Namen "Kanban" bekannten System wird auf einer Station ein Medikamentenschrank eingerichtet, welcher für die Benutzung mit "Kanban" optimiert ist. Der Schrank hat spezielle "Kanban"-Einsätze, um die "Kanban"-Behälter optimal einzuräumen. Die "Kanban"-Behälter werden in zwei Reihen eingeräumt. Das Pflegepersonal verbraucht solange Tabletten vom vorderen "Kanban"-Behälter, bis dieser leer ist. Dann wird der leere "Kanban"-behälter in die Spitalapotheke zum Nachfüllen geschickt und der hintere "Kanban"-Behälter wird zum vordern. Durch dieses System werden Unterbrüche in der Versorgung mit Medikamenten vermieden, da zu jedem Zeitpunkt jedes Medikament vorhanden ist. Das Auffüllen der "Kanban"-Behälter in der Spitalapotheke erfolgt durch Entnahme der Medikamente aus der originalen Sekundärverpackung und dem Umfüllen in den Kanban-Behälter, wobei, wenn vorhanden die Primärverpackung (beispielsweise ein Blister mit 12 Tabletten) erhalten bleibt. Die Kanban-Behälter werden mit Teilen der Originalverpackung beschriftet, damit die Medikamente erkennbar bleiben. Jeder Behälter wird mit dem Ablaufdatum, der Füllmenge, dem Bestand und einem Barcode zur Identifikation des Medikamentes beschriftet.

Es wurde wissenschaftlich festgestellt, dass etwa 10% der Fehler im Medikationsprozess beim Richten respektive Rüsten der Medikamente entstehen. Etwa 40% der Rüstfehler entstehen aufgrund von Verwechslung von Medikamenten, wie beispielsweise durch Fehler beim Ablesen, bei Medikamenten mit ähnlichem Namen oder bei Medikamenten mit ähnlichem Aussehen. Die grösste Fehlerquelle von etwa 50% besteht jedoch beim Umfüllen der Medikamente von den Verpackungen in den Dispenser. Die beim Richtprozess eingesetzten elektronisch zugriffsgeschützten Schubladensysteme und das "Kanban"-System ergeben keine merkliche Verbesserung der Fehlerquote beim Umfüllen der Medikamente.

### Darstellung der Erfindung

Aufgabe der Erfindung ist es, ein dem eingangs genannten technischen Gebiet zugehörende Vorrichtung zur Unterstützung eines manuellen Richtens von Medikamenten durch das Pflegepersonal zu schaffen, welche Fehler beim Richten von Medikamenten vermindert. Es ist eine weitere Aufgabe der Erfindung, ein System zum Richten und Verteilen von Medikamenten, ein Verfahren zum Betreiben eines solchen Systems sowie ein Computerprogrammprodukt zum Steuern eines solchen Systems zu schaffen, welche Fehler beim Richten und Verteilen von Medikamenten vermindern.

Die Lösung der Aufgabe ist durch die Merkmale des Anspruchs 1, des Anspruchs 7, des Anspruchs 14 und des Anspruchs 15 definiert.

Gemäss der Erfindung umfasst die Vorrichtung zur Unterstützung eines manuellen Richtens von Medikamenten eine Halterung für einen Medikamentenabgabebehälter, welcher wahlweise in ein oder mehrere Fächer unterteilbar ist (z.B. "WIEGAND MediDispenser"). Die Vorrichtung umfasst ferner ein elektronisch ansteuerbares Fachzeigemittel zum visuellen Hinweisen auf das zu befüllende Fach des Medikamentenabgabebehälters, und ein Steuermodul zur Steuerung des Fachzeigemittels aufgrund von vorgegebenen Verschreibungsdaten.

Die Vorrichtung zur Unterstützung eines manuellen Richtens von Medikamenten kann über Netzwerkschnittstellen mit einem Patienteninformationssystem verbunden sein, damit über entsprechende Schnittstellen Verschreibungsdaten übertragen werden können, welche insbesondere die für einen Patienten verschriebenen Medikamente umfassen.

Beim manuellen Richten von Medikamenten werden Medikamente einem Medikamentenvorrat entnommen, welcher insbesondere Medikamentenschachteln oder -packungen umfassen kann, wie sie vom Hersteller direkt ausgeliefert werden. Der Medikamentenvorrat kann aber auch spezielle Medikamentenbehälter oder -gebinde umfassen, in welche die Medikamente z.B. in einer Spitalapotheke umgefüllt werden oder welche spezielle Merkmale aufweisen, um z.B. ein Medikament luft- oder feuchtigkeitsgeschützt zu lagern. Der Medikamentenvorrat kann ferner in einem elektronisch gesteuerten System angeordnet sein, bei welchem Medikamente in elektronisch gesteuerten Schubladen angeordnet sind, welche aufgrund von Verschreibungsdaten geöffnet elektronisch geöffnet werden, damit eine Pflegeperson die für einen Patienten vorgesehenen Medikamente entnehmen und zusammenstellen kann.

Der Medikamentenabgabebehälter ist einem Patienten zugeordnet und weist mehrere Fächer auf, wobei ein Fach die Medikamente enthält, welche zu einem bestimmten Zeitpunkt, also z.B. am Morgen, am Mittag, am Abend oder in der Nacht einem Patienten abgegeben werden müssen. Der Medikamentenabgabebehälter weist also in diesem Fall dementsprechend z.B. vier Fächer auf. Die Anzahl Fächer kann aber je nach Anwendung kleiner oder grösser sein.

Die Vorrichtung zur Unterstützung eines manuellen Richtens von Medikamenten ermöglicht ein kontrolliertes manuelles Richten von Medikamenten, indem beim Richten mit den elektronisch ansteuerbaren Fachzeigemittel angezeigt wird, in welches Fach das Medikament zu füllen ist. Durch diese Unterstützung können Fehler beim Richten vermindert werden. Die erfindungsgemässe Vorrichtung ist halbautomatisch und sehr platzsparend. Bestehende Vorrichtungen und Systeme zur Lagerung von Medikamenten auf der Station können sehr einfach mit der erfindungsgemässen Vorrichtung nachgerüstet werden. Die bekannten vollständig automatisierten Systeme sind auch sehr inflexibel oder - sofern Flexibilität gegeben ist - sehr komplex und teuer. Die erfindungsgemässe Vorrichtung baut auf der bewährten Infrastruktur eines Stationszimmers in einem Spital oder Pflegeheim auf. Sie unterstützt das Richten von Arzneimitteln aller galenischer Formen wie auch von Pflegematerial. Sie passt sich in die bewährten organisatorischen Abläufe ein und schafft dadurch zusätzlich Sicherheit bei der Medikamentenversorgung der Patienten. Indem die Pflegefachperson beim Richten der Medikamente involviert ist, kann sie ihr Wissen weiter pflegen und erweitern. Die Gefahr, dass von ungenügend vorgebildeten Hilfspersonen Medikamente abgegeben werden, nur weil sie im gerichteten Medikamentenabgabebehälter enthalten sind, wird deutlich reduziert. Mit der erfindungsgemässen Vorrichtung kann das Richten und das Verteilen von unterschiedlichen Pflegefachpersonen durchgeführt werden, was eine zusätzliche (menschliche, intelligente) Kontrolle ermöglicht.

Die Halterung für den zu befüllenden Medikamentenabgabebehälter definiert insbesondere die Lage des Medikamentenabgabebehälters relativ zum Fachzeigemittel. Die Halterung kann als Adapter ausgeführt sein, sodass die Halterung beispielsweise für Medikamentenabgabebehälter unterschiedlicher Grösse ausgetauscht werden kann. Die Halterung kann einfach bei einem schon bestehenden System zum Richten von Medikamenten angebracht werden, beispielsweise an einem bestehenden Schrank auf einer Krankenstation.

Die Fachzeigemittel sind insbesondere eingerichtet, um durch Lichtmittel auf ein Fach hinzuweisen, d.h. um die Aufmerksamkeit der Pflegeperson auf ein bestimmtes Fach zu richten, indem auf das Fach durch Licht mit einer speziellen Farbe in der Nähe eines Fachs oder durch eine direkte Beleuchtung eines Fachs hingewiesen wird.

Das Steuermodul zur Steuerung des Fachzeigemittels kann beispielsweise als Softwaremodul eines schon bestehenden Computers ausgeführt sein, wobei mit einer entsprechenden Steuerleitung wie einer seriellen Datenleitung eine Steuerverbindung zwischen dem Steuermodul und dem Fachzeigemittel besteht. Dadurch kann die erfindungsgemässe Vorrichtung einfach bei einem schon bestehenden computerbasierten System zum Richten von Medikamenten nachgerüstet werden.

In einer Ausführungsvariante umfasst das Fachzeigemittel einen Bildschirm, welcher benachbart zur Halterung für den Medikamentenabgabebehälter angebracht ist, um mit mindestens einem der Halterung benachbarten Bildschirmbereich auf mindestens ein Fach des Medikamentenabgabebehälters hinzuweisen. Der Bildschirm kann in einer Arbeitsplatte eingebaut sein, sodass der Bildschirm im Wesentlichen einen Teil der Oberfläche der Arbeitsplatte bildet. Die Halterung für den Medikamentenabgabebehälter kann direkt neben dem Bildschirm in die Arbeitsplatte eingebaut sein.

Das Steuermodul kann als eine Software zur Steuerung des Bildschirms ausgeführt sein, und somit durch eine entsprechende Farbgebung, Helligkeit, Blinken oder anderen visuellen Effekt eines einem Fach nahe liegenden Bildschirmbereichs auf dieses Fach des Medikamentenabgabebehälters hingewiesen wird. Dadurch kann z.B. das Befüllen erleichtert werden oder es kann ein beim Befüllen entdeckter Fehler angezeigt werden. Der Hinweis auf das zu füllende Fach kann durch die dynamische Wahl von Gestaltungsmitteln auf dem Bildschirm zusätzlich verdeutlicht werden. Zum Zeitpunkt, bei welchem die Pflegeperson Medikamente in den Medikamentenabgabebehälter legen soll, kann beispielsweise der Bildschirm dunkel geschaltet sein, bis auf den Bereich welcher auf das zu füllende Fach hinweist. Dieser Bereich kann durch eine Variation der Helligkeit oder durch ein Blinken zusätzlich hervorgehoben werden. Schliesslich können im Bildschirmbereich, welcher auf das zu füllende Fach hinweist, zusätzliche Angaben wie z.B. die Anzahl der zu füllenden Medikamente oder ein Bild der zu füllenden Medikamente angezeigt werden, sodass es für die Pflegeperson zusätzlich leichter fällt, Fehler beim Richten der Medikamente zu vermeiden.

Die Halterung wird in der Regel unmittelbar (d.h. abstandslos) neben dem Bildschirm angeordnet. Sie kann z.B. als Vertiefung ausgebildet sein, in welche der Medikamentenabgabebehälter eingesetzt wird, so dass er nicht über die Oberfläche der Arbeitsplatte vorsteht. Dies ist aber nicht zwingend. Es kann auch ein Abstand zwischen Halterung und Bildschirm vorhanden sein. Solange die visuellen Hinweise auf dem Bildschirm einem bestimmten Fach des Behälters zugeordnet werden können, liegt die Beabstandung auch noch im Rahmen der Erfindung. Es kann auch von Vorteil sein, wenn der Behälter mit seinem Rand über die Oberfläche der Arbeitsplatte vorsteht, damit nicht unbeachtet Medikamente in ein Fach gleiten können. Es ist auch denkbar, die Halterung über dem Bildschirm anzuordnen. Ist der Medikamentenbehälter beispielsweise transparent, kann die erforderliche Befüllung quasi "im Behälter" visualisiert werden, indem der Bildschirm, den entsprechenden Bereich unter dem Behälter visuell hervorhebt (Farbe, Beleuchtung, Blinken etc.).

In einer Ausführungsvariante umfasst das Fachzeigemittel ein Beleuchtungsmittel, um mindestens ein Fach des Medikamentenabgabebehälters zu beleuchten und damit darauf hinzuweisen. Das Beleuchtungsmittel kann insbesondere als ein an einem oberen Bereich eines Schranks angebrachtes Lasergerät ausgeführt sein, sodass die zu befüllenden Fächer des Medikamentenabgabebehälters mit einem Laserstrahl beleuchtet und visualisiert werden können. Durch die Beleuchtung von Fächern wird auf eine visuell sehr deutlich erkennbare Weise auf die Fächer hingewiesen, sodass Fehler beim Richten von Medikamenten vermindert werden können.

Bevorzugt umfasst die Vorrichtung zur Unterstützung eines manuellen Richtens von Medikamenten ein Lesegerät zum Erfassen von maschinenlesbaren Codes, wobei das Lesegerät eingerichtet ist, um erfasste Codes an ein Codeerfassungsmodul zur Auswertung zu übermitteln. Maschinenlesbare Codes können insbesondere Medikamentencodes, Abgabebehältercodes, Patientencodes, Personalcodes und Richtcodes umfassen. Medikamentencodes können insbesondere an einer Medikamentenschachtel oder -behälter angebracht sein. Abgabebehältercodes und/oder Richtcodes sind entsprechend an Medikamentenabgabebehälter angebracht. Patientencodes und Personalcodes sind den Patienten respektive Pflegepersonen zugeordnet und können auf Patientenblättern oder Personalausweisen angebracht sein. Der Richtcode identifiziert den Datensatz, der beim Befüllen des Medikamentenabgabebehälters geschrieben/verwendet wurde.

Diese Codes können in einem ein- oder zweidimensionalen Barcode (1 D oder 2D Barcode), einem RFID-Chip (Radio Frequency Identification), einem Magnetstreifen oder irgendeinem anderen Speichermedium abgespeichert sein.

Dementsprechend umfasst das Lesegerät Mittel, um maschinenlesbare Codes mit einer optischen Vorrichtung und/oder mittels Radiowellen zu erfassen. So können eindimensionale oder zweidimensionale Barcodes mittels eines Lesegeräts erfasst werden, welches mit einem Laserabtastgerät oder einer Kamera ausgerüstet ist. Daten eines RFID-Chips können mit einem Lesegerät erfasst werden, welches einen entsprechenden Radiowellensender und -empfänger aufweist. Selbstverständlich kann das Lesegerät als ein kombiniertes Lesegerät aufgebaut sein, sodass beispielsweise mit demselben Lesegerät sowohl Daten von 1 D/2D Barcodes als auch Daten von RFID-Chips erfasst werden können.

Das Lesegerät kann weiter Mittel umfassen, um maschinenlesbare Codes mit einer Vorrichtung zur Erfassung von magnetischen Feldern zu erfassen. Somit können Daten eines Magnetstreifens mit einem Lesegerät erfasst werden, welches einen magnetischen Lesekopf aufweist.

Das Lesegerät zur Erfassung der maschinenlesbaren Codes ist beispielsweise in eine Arbeitsplatte einer Medikamentenrichtstation (z.B. flächenbündig) eingebaut. Dadurch wird die Handhabung einer solchen Medikamentenrichtstation zusätzlich erleichtert, da es oft sowieso notwendig ist, eine Medikamentenschachtel vor der Entnahme von Medikamenten abzustellen, um die Schachtel aufzumachen und Medikamente zu entnehmen. So kann der maschinenlesbare Medikamentencode einer Medikamentenschachtel unmittelbar beim Öffnen erfasst werden und es kann überprüft werden, dass das korrekte Medikament ausgewählt wurde. Falls festgestellt wird, dass die Pflegeperson eine falsche Medikamentenschachtel ausgewählt hat, kann dies zusätzlich durch ein akustisches Signal angezeigt werden, da die Pflegeperson zu diesem Zeitpunkt die Aufmerksamkeit auf das Auspacken der Medikamentschachtel gelenkt hat und bei einer Anzeige auf einem Bildschirm ggf. ungenügend über das Missgeschick informiert würde. Das Lesegerät zur Erfassung der maschinenlesbaren Codes kann alternativ als Handscanner ausgeführt sein, welcher über eine drahtlose oder drahtbasierte Datenverbindung mit dem Codeerfassungsmodul verbunden ist. Es ist auch möglich, das Lesegerät auf oder oberhalb der Arbeitsplatte zu fixieren. Als Vorbild können Anordnungen dienen, wie sie bei modernen Registrierkassen in Einkaufszentren zur Anwendung kommen.

Das Codeerfassungsmodul kann beispielsweise als Softwaremodul eines schon bestehenden Computers ausgeführt sein, wobei eine Datenleitung als Verbindung zwischen dem Codeerfassungsmodul und dem Lesegerät besteht. Dadurch kann die erfindungsgemässe Vorrichtung einfach bei einem schon bestehenden computerbasierten System zum Richten von Medikamenten nachgerüstet werden. Das Codeerfassungsmodul kann mit weiteren Softwaremodulen zusammenwirken, sodass beim Einlesen eines Personalcodes eine Freischaltung für den Gebrauch der Vorrichtung erfolgt. Beim Einlesen eines Abgabebehältercodes werden beispielsweise die Verschreibungsdaten eines Patienten aus einer Datenbank abgerufen und das Richten von Medikamenten wird für diesen Patienten ausgelöst, indem beispielsweise auf einem Bildschirm ein Medikament angezeigt wird und durch ein Zusammenwirken des Codeerfassungsmoduls mit dem Steuermodul durch die Fachzeigemittel auf das Fach hingewiesen wird, in welches das Medikament zu füllen ist. Beim Einlesen eines Medikamentencodes wird überprüft, ob dieses Medikament für den aktuellen Patienten vorgesehen ist und es wird gegebenenfalls eine akustische oder optische Warnung ausgelöst. Beim Einlesen eines Patientencodes wird beispielsweise das Richten von Medikamenten für einen weiteren Patienten ausgelöst.

Vorzugsweise umfasst die Vorrichtung zur Unterstützung eines manuellen Richtens von Medikamenten eine Einfüllkontrolleinheit, welche insbesondere eine Lichtschranke, eine Foto-/Videokamera und/oder ein Distanzmessgerät aufweist, um das Einfüllen eines Medikaments in ein Fach des Medikamentenabgabebehälters zu überwachen. Die Einfüllkontrolleinheit umfasst eine Signalisierungseinrichtung, welche das Überwachungsresultat an ein Einfüllkontrollmodul übermittelt, wobei insbesondere ein Bild des fertig gerichteten Medikamentenabgabebehälters zur Dokumentation übermittelt wird.

Die Einfüllkontrolleinheit kann als Lichtschranke mit linienförmigen Elementen gebildet sein, welche die gesamte Fläche eines Fachs des Medikamentenabgabebehälters überwachen und so das Hineinlegen eines Medikaments in das Fach zuverlässig detektieren. Falls mehrere Einheiten eines Medikaments in dasselbe Fach abgelegt werden müssen, kann durch ein entsprechenden Hinweis am Bildschirm darauf hingewiesen werden, dass diese Nacheinander in das Fach gelegt werden müssen, um die Anzahl zuverlässig zu erfassen. Alternativ kann die Halterung für den Medikamentenabgabebehälter eine Präzisionswaage umfassen, sodass bei jedem Arbeitsschritt durch ein Wägen festgestellt werden kann, ob das erforderliche Medikament in ein Fach gelegt wurde. Selbstverständlich können die Lichtschranke und die Präzisionswaage kombiniert eingebaut sein, sodass mit der Lichtschranke detektiert wird, in welches Fach ein Medikament abgelegt wurde und mit der Präzisionswaage überprüft wird, dass die korrekte Anzahl Medikamente abgelegt wurde.

Alternativ kann die Einfüllkontrolleinheit als Foto- und/oder Videokamera ausgeführt sein, welche an einem hinteren Teil der Arbeitsfläche angeordnet ist und den Bereich um den in der Halterung angeordneten Medikamentenabgabebehälter vollständig überwacht, sodass festgestellt werden kann, in welches der Fächer hineingegriffen wird. Ebenso wäre es möglich mit Hilfe eines seitlich installierten Distanzsensors festzustellen, an welcher Stelle in ein Fach hineingegriffen wird. Falls eine solche Foto- und/oder Videokamera vorgesehen ist, dann kann diese auch als Lesegerät zum Einlesen von Barcodes verwendet werden.

Die Einfüllkontrolleinheit umfasst bevorzugt eine Fotokamera, um ein Bild des befüllten Medikamentenabgabebehälters zur Dokumentation zu erfassen. Falls schon eine Foto-und/oder Videokamera zur Überwachung des Einfüllens angebracht ist, kann diese zur Dokumentation verwendet werden. So kann es beispielsweise vorgesehen sein, dass der Medikamentenabgabebehälter nach dem Befüllen wie an anderer Stelle beschrieben mit einer Etikette neu beschriftet wird und deshalb eine Etikette gedruckt werden muss. Zusammen mit dem Drucken der Etikette kann ein Bild des Medikamentenabgabebehälters erfasst und in einer Datenbank abgespeichert werden. Reagiert ein Patient bei der Medikamenteneinnahme ungewöhnlich, dann kann so durch eine entsprechende Datenbankabfrage rasch überprüft werden, ob eine Verwechslung der Medikamente ausgeschlossen werden kann. Es kann auch optisch überprüft werden, was gerichtet wurde. Die Verwechslung der Medikamente wird bereits beim Richten überprüft.

Bevorzugt ist/sind der Bildschirm, das Lesegerät, die Halterung und/oder die Einfüllkontrolleinheit in eine Medikamentenrichtstation einbaubare Arbeitsplatte eingebaut und/oder daran angebracht, wobei insbesondere eine Glasplatte vorgesehen ist, welche die Arbeitsplatte im Wesentlichen ganzflächig abdeckt. Alternativ ist/sind der Bildschirm, das Lesegerät, die Halterung und/oder die Einfüllkontrolleinheit in eine Medikamentenrichtstation auf irgendeine andere hygienische Art eingebaut und/oder daran angebracht. Vorzugsweise ist die Arbeitsplatte ausziehbar an einem Schrank angeordnet. Bei Nichtgebrauch der Vorrichtung kann so die Arbeitsplatte vollständig im Schrank versorgt werden und durch einen abschliessbaren Laden oder eine abschliessbare Tür kann der unbefugte Zugriff auf Medikamente und/oder die Arbeitsplatte unterbunden werden. Alternativ ist die Arbeitsplatte ausklappbar, ausschwenkbar oder faltbar ausgebildet, sodass beispielsweise bei eingeklappter Arbeitsplatte der Schrank durch die Arbeitsplatte mindestens teilweise verschlossen wird.

Bevorzugt ist auf einer Oberseite der Arbeitsplatte eine ganzflächige Glasplatte vorgesehen, welche auch den Bildschirm abdeckt. Dadurch kann der Arbeitsbereich einfach gereinigt werden und hygienisch sauber gehalten werden, da die Glasfläche auch bei aggressiven Reinigungs- oder Desinfektionsmittel nicht angegriffen wird. Alternativ weist die Arbeitsplatte auf der Oberseite eine Kunststoffbeschichtung auf, welche bei Verschmutzung ausgetauscht wird. Es ist z.B. denkbar, dass der Bildschirm in der Arbeitsplatte schwenkbar gelagert ist, so dass er entweder horizontal in der Arbeitsplatte liegen kann oder in eine geeignete Schrägstellung geschwenkt werden kann. In einer solchen Ausführung macht eine durchgehende Glasabdeckung jedoch keinen Sinn. Es reicht aus, wenn Bildschirm einerseits und umgebende Arbeitsplatte andererseits je mit einer reinigungsfreundlichen Oberfläche versehen sind.

Ein System zum Richten und Verteilen von Medikamenten umfasst eine Medikamentenrichtstation mit einer Vorrichtung zur Lagerung eines Medikamentenvorrats, insbesondere einem Schrank (oder z.B. einer Schubladensäule), eine beschriebene Vorrichtung zur Unterstützung des manuellen Richtens von Medikamenten und eine elektronische Schaltung, welche das Steuermodul und gegebenenfalls das Codeerfassungsmodul und/oder die Einfüllkontrolleinheit umfasst. Das System umfasst weiter ein Eingabegerät zur manuellen Steuerung der elektronischen Schaltung. Insbesondere zur Überwachung der manuellen Steuerung umfasst das System bevorzugt einen Bildschirm.

Ein Schrank zur Lagerung eines Medikamentenvorrats ist beispielsweise als kostengünstiger Standardschrank oder Einbauschrank mit geeigneter Innenausstattung ausgeführt. Die Innenausstattung kann z.B. Auszugstablare, Auszugskörbe, Schachtelspender (bei welchen eine Reihe von Schachteln bereitgestellt ist und beim Entnehmen der vordersten Schachtel die hinteren nachgeschoben werden) oder einfach fixe Fächer umfassen. Der Schrank muss zum Arbeiten vollständig geöffnet werden können, so dass die Bedienperson beim Richten freien Zugang zum gesamten Schrankinhalt (insbesondere zum gesamten Medikamentenvorrat) hat. Idealerweise haben die Schränke im oberen Bereich Rollladen. Meistens werden 3-5 Schränke nebeneinander gestellt. Aus Kostengründen wird oft nur der mittlere Schrank mit einem Rollladen ausgerüstet und die äusseren mit Flügeltüren, die sich nach aussen öffnen. So stehen der Pflegeperson beim Richten keine Türen im Wege, es können alle Türen gleichzeitig geöffnet sein, um den Schränken Medikamente und Materialien zu entnehmen. Es sind auch Schiebetüren oder mechanische Vorrichtungen denkbar, soweit sie es zulassen, dass der ganze, für das Richten benötigte Medikamentenvorrat frei (gleichzeitig) zugänglich ist.

Die Medikamente können in den handelsüblichen Schachteln oder Packungen (wie sie von den Medikamentenherstellern an Apotheken und Ärzte abgegeben werden) oder in "Kanban"-Behältern im Schrank gelagert sein. Auch die angebrauchten Schachteln oder Packungen werden im Schrank gelagert. Die Medikamente können auch in einem Schrank mit elektronisch gesteuerten Schubladen angeordnet sein. Beim Richten von Medikamenten werden die Schubladen entsprechend Verschreibungsdaten elektronisch geöffnet.

Die elektronische Schaltung schafft den funktionellen Zusammenhang, der letztendlich die höhere Zuverlässigkeit beim Richten gewährleistet. Sie ist typischerweise durch eine handelsübliche Computerhardware in Verbindung mit einer spezifischen Software realisiert. Sie verfolgt und kontrolliert den Richtvorgang auf der Basis der Eingabe (Lesegerät zum Erfassen des Medikaments, Befüllkontrolle des Medikamentenabgabebehälters, Tastatur etc.) und der Ausgabegeräte (Bildschirm, akustische Ausgabe oder ähnlichem). Die Software greift auf die Patientendaten zu und speichert die Eingaben des Bedienpersonals ab. Auf diese Weise ist eine spätere Kontrolle des Ablaufs gewährleistet, sofern sich die Frage nach einem Fehler stellen sollte.

Bevorzugt umfasst der Medikamentenvorrat mehrere Medikamentenbehälter mit je einem daran angebrachten maschinenlesbaren Medikamentencode zur Identifikation eines Medikaments. Die Medikamentenbehälter können handelsübliche Medikamentenschachteln umfassen. Dadurch ist ein Umverpacken, wie es in bekannten vollautomatischen Systemen erforderlich ist, nicht nötig.

Alternativ werden die handelsüblichen Schachteln in einer zentralen Apotheke in die erwähnten "Kanban"-Behälter umverpackt. Dadurch steigt die Übersicht in den im Schrank angeordneten Medikamenten und der Rüstprozess wird schneller, da sich die "Kanban"-Behälter leichter öffnen und schliessen lassen.

Bevorzugt umfasst das System zum Richten und Verteilen von Medikamenten eine Mehrzahl von wahlweise in ein oder mehrere Fächer unterteilbare Medikamentenabgabebehälter mit daran angebrachtem maschinenlesbaren Abgabebehältercode und/oder Richtcode. Den Abgabebehältercodes und/oder Richtcodes sind insbesondere Patientendaten respektive Verschreibungsdaten zugeordnet. Ein Lesegerät ist eingerichtet, um die maschinenlesbaren Abgabebehältercodes und/oder Richtcodes zu erfassen. Der Abgabebehältercode und/oder Richtcode können beispielsweise zusammen mit einen Patientennamen und Geburtsdatum auf dem Medikamentenabgabebehälter aufgeklebt sein und einen ein-oder zweidimensionalen Barcode (1 D oder 2D Barcode) umfassen. Alternativ kann am Medikamentenabgabebehälter ein RFID-Chip (Radio Frequency Identification) angeordnet sein, wobei der Abgabebehältercode und/oder der Richtcode auf dem RFID-Chip abgespeichert sind oder ein auf dem RFID-Chip abgespeicherter Code einem Abgabebehältercode und/oder Richtcode zugeordnet werden kann. Der Abgabebehältercode des Medikamentenabgabebehälters kann z.B. vor dem Einsetzen des Medikamentenabgabebehälters in die Halterung mit dem Lesegerät erfasst werden.

Alternativ kann ein Abgabebehältercode, ein Patientencode oder Patientendaten am Bildschirm eingegeben werden. Mit einer entsprechenden Datenbankabfrage der Verschreibungsdaten (entweder lokal oder über ein Netzwerk) können auf dem Bildschirm in einer Liste die Medikamente angegeben werden, welche für den Patienten zu richten sind.

Vorzugsweise sind die Anzahl und/oder die Grösse der Fächer des Medikamentenabgabebehälters einstellbar. Der Medikamentenbehälter kann z.B. ein rechteckiger formstabiler Kunststoffbehälter sein, welcher mit diversen Rillen an der Innenseite ausgebildet ist, so dass mit dem Einfügen von formstabilen Kunststoffplättchen eine Unterteilung des Innenraums geschaffen wird (die Rillenanordnung definiert die verfügbare Aufteilung des Innenraums). Selbstverständlich sind andere Arten denkbar, um die Grösse und/oder die Anzahl Fächer einzustellen, so können die Fächer z.B. als Einheiten unterschiedlicher Grösse aufgebaut sein, welche sich zu einem Medikamentenabgabebehälter zusammenstecken lassen. Es wäre auch möglich, einen Medikamentenabgabebehälter mit einer schiebbaren Unterteilung der Fächer zu verwenden. Dadurch lässt sich der Medikamentenabgabebehälter an die Bedürfnisse eines Patienten anpassen, sodass bei einer grossen Anzahl oder voluminösen Medikamenten genügend Platz vorhanden ist. Selbstverständlich können die Bereiche auf dem Bildschirm, welche auf das zu befüllende Fach hinweisen, entsprechend angepasst werden, indem die Grösse der eingestellten Fächer erfasst und mit der elektronischen Schaltung die Anzeigebereiche auf dem Bildschirm dynamisch angepasst werden. Durch die Wahl der Grösse der Fächer des Medikamentenabgabebehälter eignet sich dieser für unterschiedliche Arzneimittel wie Tabletten, Suppositorien, Sachets, Ampullen, etc. und es lassen sich unter Umständen auch Hilfsmittel wie Becher, Einwegspritzen, Nadeln, etc. geeignet richten, lagern und verteilen.

Bevorzugt ist der Innenraum des Medikamentenabgabebehälters entsprechend der möglichen Aufteilung visuell markiert (z.B. durch grafische Markierung wie Farbe oder Beschriftung, oder durch Oberflächenkonturierung). Die visuelle Markierung erlaubt es dem Bedienpersonal die einzelnen Fächer im Fall einer Unterteilung in zwei oder mehrere Fächer zu identifizieren. Zugleich kann diese Markierung von der Software dazu verwendet werden, die Bildschirmanzeige entsprechend zu steuern. Dazu kann die Einteilung der Fächer in der Software konfiguriert werden. Mit anderen Worten: Der Medikamentenabgabebehälter kann (wie bereits weiter oben erwähnt) vom Bedienpersonal in eine gewünschte Anzahl Fächer unterteilt werden und jedes dieser Fächer kann über die visuelle Markierung identifiziert werden.

Wenn die Fächer des Medikamentenabgabebehälters z.B. unterschiedliche Farben aufweisen, kann auf dem Bildschirm zum Befüllen eines bestimmten Fachs ein dem Fach benachbarter Bereich mit einer entsprechenden Farbe signalisiert werden. Durch eine farbliche Kennzeichnung kann die Verwechslungsgefahr der Fächer erheblich vermindert und somit die Fehler bei der Medikamentenabgabe zusätzlich verkleinert werden. Die Fächer können mit Farben, mit Beschriftung ("Morgen", Mittag", "Abend", "Nacht"), mit Abgabezeiten wie "12:00", mit Nummern, Buchstaben oder anderen Zeichen markiert sein, wobei auf zugeordneten Bildschirmbereichen eine jeweils entsprechende Darstellung angezeigt wird.

Bevorzugt umfasst das Eingabegerät einen berührungsempfindlichen Bildschirm. Dann kann zur Steuerung der Vorrichtung und zur Eingabe von Daten auf eine Tastatur verzichtet werden, wodurch eine flexible und dynamisch anpassbare Eingabe von Daten ermöglicht wird. Für Sonderaufgaben wie beispielsweise das Einspielen von Softwareupdates können jedoch im Schrank zusätzlich eine Tastatur und eine Maus gelagert sein.

Die Eingabe kann auch über einen separaten berührungsempfindlichen Bildschirm erfolgen. Dies kann von Vorteil sein, wenn damit die Bedienbarkeit während des Richtens verbessert wird. Insbesondere kann so der als Eingabe funktionierende Bildschirm schräg gestellt werden (was bei einer Integration in der Arbeitsplatte weniger gut möglich ist, da eine Arbeitsfläche in aller Regel mehr oder weniger horizontal angeordnet sein soll).

Vorzugsweise ist eine Kennzeichnungseinheit vorgesehen, um maschinenlesbare Codes wie Abgabebehältercodes, Richtcodes und Arzneimittelcodes zu erzeugen. Der Abgabebehälter ist mit einem kombinierten Code, einem Abgabebehältercode oder einem Richtcode ausgerüstet, um ihn einem Patienten und dessen Verschreibungsdaten zuzuordnen. Einzelgerichtete Medikamente sind mit einem kombinierten Code oder einem Richtcode ausgerüstet um sie einem Patienten und dessen Verschreibungsdaten zuzuordnen. Der kombinierte Code umfasst unter anderem z.B. einen Patienten-, einen Medikamenten- und/oder einen Richtcode. Zur Zuordnung eines Abgabebehälters oder eines einzelgerichteten Medikamentes zu Patienten und deren Verschreibungsdaten werden die oben beschriebenen Codes mit dem Lesegerät erfasst und mit Hilfe einer Datenbank zu Patienten- und Verschreibungsdaten zugeordnet. Die Kennzeichnungseinheit kann weiter einen Etikettendrucker umfassen, welcher eingerichtet ist, um 1 D und/oder 2D Barcodes auf Etiketten zu drucken. Alternativ kann die Kennzeichnungseinheit ein RFID Gerät umfassen, mit welchem RFID Daten auf einen RFID-Chip geschrieben werden können. Die Kennzeichnungseinheit ist bevorzugt eingerichtet, um die kombinierten Codes, die Abgabebehältercodes und/oder Richtcodes zu Verschreibungsdaten zuzuordnen und diese Zuordnung in einer Datenbank abzuspeichern, sodass bei einem erneuten Einlesen des Codes Verschreibungsdaten eines Patienten eindeutig ermittelt werden können.

Vorzugsweise ist eine mobile Verteileinrichtung vorgesehen zur Aufnahme und zum Transport von Medikamentenabgabebehältern, wobei die mobile Verteileinrichtung eine Anzeigeeinheit, eine Eingabeeinheit und ein Lesegerät zur Erfassung von maschinenlesbaren Codes aufweist. Die mobile Verteileinrichtung kann in der Art eines Rollkorpus ausgeführt sein, welcher von der Pflegeperson von Zimmer zu Zimmer geschoben werden kann, wenn die gerichteten Medikamente abgegeben werden. Die mobile Verteileinrichtung kann auch als tragbare Einrichtung ausgestaltet sein, welche von der Pflegeperson von Zimmer zu Zimmer mitgetragen wird.

In der mobilen Verteileinrichtung können die Medikamentenabgabebehälter auf ausziehbaren Tablaren angeordnet sein. Die Anzeigeeinheit und Eingabeeinheit kann durch einen Computer wie einen Laptop mit einem Touchscreen oder durch ein anderes mobiles Gerät gebildet sein. Das Lesegerät zur Erfassung von maschinenlesbaren Medikamenten-und/oder Abgabebehältercodes kann beispielsweise als Handscanner ausgeführt und mit dem Computer über eine Datenverbindung verbunden sein. Daten betreffend Patienten und Medikamentenabgabebehälter sind auf dem Laptop gespeichert oder über eine Datenleitung wie eine drahtlose Datenleitung aus einer Datenbank abrufbar.

Somit kann eine Pflegeperson mit der mobilen Verteileinrichtung zu einem Bett eines Patienten gehen und auf dem Laptop die Daten dieses Patienten selektieren. Auf der Anzeigeeinheit und Eingabeeinheit wird der Medikamentenabgabebehälter angezeigt, beispielsweise indem eine Tablarnummer und eine Behälternummer angezeigt werden. Die Pflegeperson entnimmt den Medikamentenabgabebehälter der mobilen Verteileinrichtung und liest mit dem Lesegerät den daran angebrachten Abgabebehältercode ein. Nach einer Überprüfung des Abgabebehältercodes werden auf dem Bildschirm ein Fach des Medikamentenabgabebehälters sowie gegebenenfalls die zu verabreichenden Medikamente angezeigt. Die Pflegeperson entnimmt die Medikamente dem entsprechenden Fach und übergibt oder verabreicht diese dem Patienten. Gegebenfalls werden auf dem Bildschirm Indikationen angezeigt, welche die Pflegeperson bei oder nach der Verabreichung der Medikamente überwachen muss. Solche Indikationen können auch zu einem späteren Zeitpunkt angezeigt werden, also nachdem ein Medikament vollständig wirkt, sodass die Pflegeperson daran erinnert wird, zum Bett des Patienten zurückzukehren um die notwendigen Überwachungsschritte, wie beispielsweise das Messen einer Körpertemperatur oder die Kontrolle des Pulses, vorzunehmen.

Bevorzugt umfasst das System zum Richten und Verteilen von Medikamenten eine Mehrzahl von Patientenkennzeichnungen mit maschinenlesbarem Patientencode. Eine solche Patientenkennzeichnung kann am Bett des Patienten angebracht sein. So kann durch ein Einlesen und Überprüfen des maschinenlesbaren Patientencodes der Patientenkennzeichnung und der Codes am Abgabebehälter und/oder an einzelgerichteten Medikamenten sichergestellt werden, dass der Abgabebehälter und die einzelgerichteten Medikamente mit dem dafür vorgesehenen Patienten übereinstimmen und so eine Verwechslung ausgeschlossen werden.

In einem Verfahren zum Betreiben eines Systems zum Richten und Verteilen von Medikamenten erfolgt eine elektronische Aufforderung, ein Medikament in den Medikamentenabgabebehälter zu legen und es wird angezeigt, in welches der Fächer des Medikamentenabgabebehälters das Medikament zu füllen ist, indem mit den Fachzeigemittel auf dieses Fach hingewiesen wird.

Bevorzugt erfolgen gegebenenfalls eine elektronische Aufforderung zur Selektion von Verschreibungsdaten, eine elektronische Aufforderung zum Anordnen eines wahlweise in ein oder mehrere Fächer unterteilbaren Medikamentenabgabebehälters in der Halterung der Vorrichtung zur Unterstützung eines manuellen Richtens von Medikamenten und eine elektronische Aufforderung zur Entnahme eines Medikaments aus dem Medikamentenvorrat. Vorzugsweise erfolgt gegebenenfalls eine elektronische Aufforderung zum Einlesen eines maschinenlesbaren Medikamentencodes mit einem Lesegerät und das Einfüllen des Medikaments mit einer Einfüllkontrolleinheit wird vorzugsweise überwacht. Wird im maschinenlesbaren Medikamentencode auch die Charge oder das Verfalldatum mitgeliefert, dann kann dies dem Patienten zugeordnet werden. Es wird angezeigt, welches Medikament in welches der Fächer des Medikamentenabgabebehälters zu füllen ist, indem auf einem zum befüllenden Fach nächstliegenden Bildschirmbereich eines Bildschirms oder mit einem Beleuchtungsgerät zum Beleuchten eines Fachs auf dieses Fach hingewiesen wird. Ein solches Verfahren bietet einer Pflegeperson, welche Medikamente zum Verteilen in einem Medikamentenabgabebehälter richtet, eine exakte Unterstützung, sodass Fehler beim Richten von Medikamenten stark reduziert werden können.

Die Selektion von Verschreibungsdaten kann durch Einlesen eines Abgabebehältercodes vom Medikamentenabgabebehälter erfolgen. Stattdessen kann durch das System ein Patient automatisch vorgegeben und auf dem Bildschirm angezeigt werden, so dass die Pflegeperson, welche die Medikamente richtet, zunächst den entsprechend gekennzeichneten Medikamentenabgabebehälter oder einen ganz neuen, noch nicht gekennzeichneten Medikamentenabgabebehälter auswählt und in die Halterung für den Medikamentenabgabebehälter legt.

Bevorzugt umfasst das Verfahren einen Schritt um mit einer Kennzeichnungseinheit einen Abgabebehältercode und/oder Richtcode zu erzeugen der an einen Medikamentenabgabebehälter angebracht wird und/oder um einen an einem Medikamentenabgabebehälter angebrachten Abgabebehältercode mit einem Patientencode zu Patientendaten zuzuordnen.

Vorzugsweise umfasst das Verfahren einen Schritt um mit einer Einfüllkontrolleinheit das Einfüllen von Medikamenten in die Fächer des Medikamentenabgabebehälters zu überwachen.

Bevorzugt umfasst das Verfahren einen Schritt um mit einer Fotokamera ein Bild des befüllten und somit fertig gerichteten Medikamentenabgabebehälters zur Dokumentation zu erfassen.

Vorzugsweise wird der Medikamentenabgabebehälter in einer mobilen Verteileinrichtung zum Transport angeordnet. Mit einem Lesegerät der mobilen Verteileinrichtung wird ein Abgabebehältercode und/oder Richtcode erfasst und es wird auf einer Anzeigeeinheit der mobilen Verteileinrichtung ein Medikamentenabgabebehälter angezeigt, welcher Medikamente für den entsprechenden Patienten enthält. Durch das Einlesen eines am Medikamentenabgabebehälter angebrachten Abgabebehältercodes und/oder Richtcodes wird registriert, ob der richtige Medikamentenabgabebehälter der mobilen Einrichtung entnommen wurde. Gegebenenfalls wird eine akustische oder optische Warnung erzeugt.

Ein Computerprogrammprodukt zur Steuerung eines Systems zum Richten und Verteilen von Medikamenten umfasst eine oder mehrere der folgenden Softwaremodule. Ein Selektionsmodul zur automatischen oder manuellen Selektion von Verschreibungsdaten aus einer Datenbank mit Verschreibungsdaten. Ein Anordnungsmodul zur Aufforderung/Überwachung des Anordnens eines wahlweise in ein oder mehrere Fächer unterteilbaren Medikamentenabgabebehälters in der Halterung der Vorrichtung zur Unterstützung eines manuellen Richtens von Medikamenten. Ein Codeerfassungsmodul zum Einlesen und Erfassen von Codes mit einem Lesegerät, wie insbesondere maschinenlesbare Medikamentencodes, Abgabebehältercodes, Richtcodes, Patientencodes und Personalcodes. Ein Vergleichsmodul zum Vergleichen von eingelesenen Codes mit Verschreibungsdaten. Ein Steuermodul zum Hinweisen auf ein Fach des Medikamentenabgabebehälters mit dem Fachzeigemittel. Ein Einfüllkontrollmodul zur Überwachung des Einfüllens von Medikamenten in ein Fach des Medikamentenabgabebehälters mit einer Einfüllkontrolleinheit. Diese Funktionen können als einzelne Softwaremodule erstellt sein und in Programmiersprachen/Programmierumgebungen wie C, C++, Java, Framework 3, Visual Studio 2008® oder irgendeiner anderen Programmierumgebung abgefasst sein. Das Computerprogrammprodukt läuft insbesondere auf einem PC ab, der auch als Industrie PC ausgeführt sein kann.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine perspektivische Ansicht einer erfindungsgemässen Medikamenten- richtstation;
- Fig. 2: eine perspektivische Ansicht des Auszugtablars der Medikamentenrichtsta- tion;
- Fig. 3: eine Ansicht des im Auszugstablar eingebauten Bildschirms;
- Fig. 4: eine perspektivische Ansicht eines Medikamentenabgabebehälters;
- Fig. 5a, 5b: schematisch ein Flussdiagram einer Software zum Steuern des Richtens und Verteilens von Medikamenten;
- Fig. 6: schematisch ein Computerprogrammprodukt umfassend mehrere Soft- waremodule.

Grundsätzlich sind in den Figuren gleiche Teile mit gleichen Bezugszeichen versehen.

### Wege zur Ausführung der Erfindung

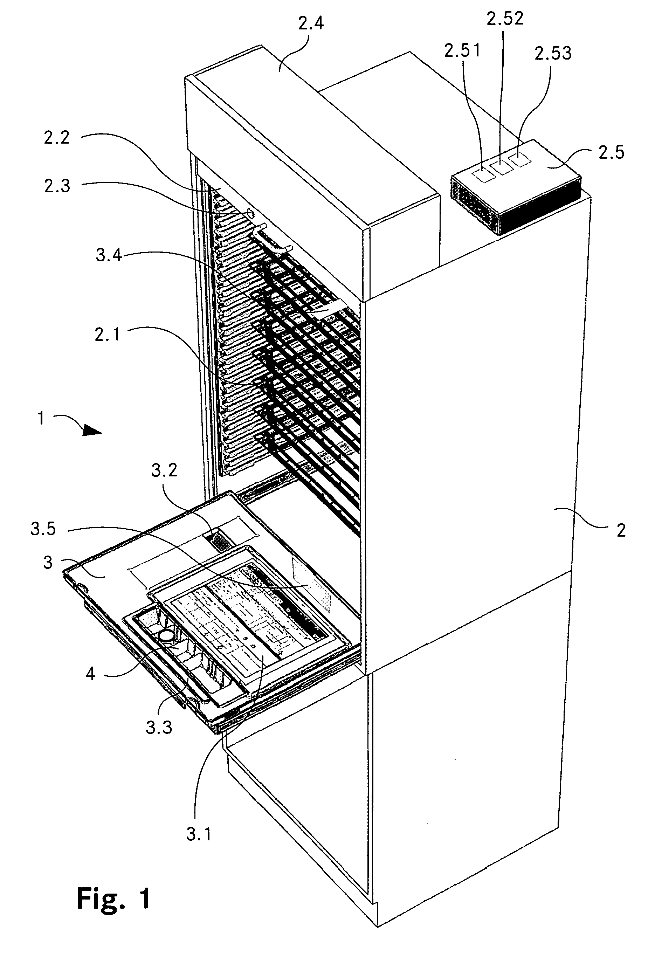

Fig. 1 zeigt die perspektivische Ansicht einer erfindungsgemässen Medikamentenrichtstation 1. Diese umfasst einen Schrank 2, welcher beispielsweise wie in Fig. 1 dargestellt zweiteilig aufgebaut ist. Beispielsweise in einem oberen Teil weist der Schrank 2 Lagertablare 2.1 auf zur Lagerung von Medikamentenschachtein. Auf den Lagertablaren 2.1 können auch weitere medizinische Produkte wie Spritzen, Messbecher, etc. angeordnet sein. Die Lagertablare 2.1 sind ausziehbar oder feststehend im Schrank 2 angeordnet. Auf einer für eine stehende oder sitzende Person angenehmen Höhe ist am Schrank 2 eine Arbeitsplatte 3 angeordnet, beispielsweise ist die Arbeitsplatte 3 am Schrank 2 ausziehbar angebracht. Der Schrank 2 weist einen Laden 2.2 auf, mit welchem der Schrank 2 geschlossen werden kann. Der Schrank 2 ist mit einem elektronischen oder mechanischen Schloss 2.3 zum Schutz vor unberechtigtem Zugriff abschliessbar. Auf dem Schrank 2 sind eine Aufnahmebox 2.4 zur Aufnahme von Teilen des geöffneten Ladens 2.2 und eine elektronische Schaltung 2.5 zur Durchführung von nachfolgend dargestellten Aufgaben und Funktionen angeordnet. In dem in Fig. 1 gezeigten Beispiel ist der Laden 2.2 in geöffnetem Zustand in der Aufnahmebox 2.4 aufgerollt. Selbstverständlich können stattdessen am Schrank 2 eine oder mehrere Schranktüren vorgesehen sein.

Die auf den Lagertablaren 2.1 angeordneten Medikamentenschachteln weisen maschinenlesbare Medikamentencodes zur Identifikation des Medikaments auf. Als maschinenlesbare Medikamentencodes können die 1 D- oder 2D-Barcodes der Originalpackungen von Medikamenten oder von Lageretiketten verwendet werden. Alternativ können zur Identifikation des Medikaments RFID-Chips (Radio Frequency Identification) respektive RFID-Transponder an den Medikamentenschachteln angebracht sein.

In der Arbeitsplatte 3 ist ein Bildschirm 3.1 eingebaut. Der Bildschirm 3.1 ist beispielsweise als erschütterungsfester 15" Projected Capacitive Touchscreen ausgeführt und weist die Anschlüsse VGA, USB/Serial-Touch und 12V Power auf. Der Touchscreen ist beispielsweise mit einer Glasplatte abgedeckt, sodass die Arbeitsfläche der Arbeitsplatte 3 einwandfrei rein gehalten werden kann. Beim Zurückfahren der Arbeitsplatte 3 wird das Backlight des Bildschirms abgeschaltet, der Touchpanel (d.h. die Tastenfunktion des Bildschirms 3.1) bleibt jedoch eingeschaltet, damit der Bildschirm ohne Verzögerung wieder bereit ist. Zum Ein-/Ausschalten des Backlight ist ein Schalter vorgesehen, welcher geeignet montiert ist, wobei der Zustand des Schalters von der elektronischen Schaltung 2.5 abfragbar ist.

An den Bildschirm 3.1 anschliessend weist die Arbeitsplatte 3 eine Halterung 3.3 zur Aufnahme eines Medikamentenabgabebehälters 4 auf. Der Medikamentenbehälter 4 weist verschiedene Fächer zur Aufnahme von Medikamenten auf und ist beispielsweise mittels eines transparenten Schiebedeckels verschliessbar, sodass die in den Fächern angeordneten Medikamente vor dem Herausfallen und vor Umwelteinflüssen wie Feuchtigkeit geschützt sind. Die Halterung 3.3 und der Medikamentenabgabebehälter 4 sind derart ausgeführt, dass eine Oberkante des Medikamentenabgabebehälters 4 an den Bildschirms 3.1 anschliesst. Der Medikamentenabgabebehälter 4 weist einen Abgabebehältercode und/oder Richtcode auf, welcher Patientendaten zugeordnet ist.

Weiter kann ein Beleuchtungsgerät 3.4 angeordnet sein, beispielsweise an einem oberen Teil des Schranks 2. Das Beleuchtungsgerät 3.4 ist insbesondere als Lasergerät ausgeführt und eingerichtet, um eines oder mehrere Fächer zu beleuchten. Zur Steuerung des Beleuchtungsgeräts 3.4 ist dieses über eine Steuerverbindung wie einer seriellen Datenverbindung mit der elektronischen Schaltung 2.5 verbunden.

Die elektronische Schaltung 2.5 umfasst insbesondere ein Steuermodul 2.51, ein Codeerfassungsmodul 2.52 und ein Einfüllkontrollmodul 2.53, welche insbesondere als auf einem Mikroprozessor ablauffähige Softwaremodule ausgeführt sein können.

An der Arbeitsplatte 3 ist ein Lesegerät 3.2 angeordnet, um maschinenlesbare Medikamenten- und/oder Abgabebehältercodes wie 1D- oder 2D-Barcodes und/oder auf RFID-Chips abgespeicherte Codes zu erfassen. Das Lesegerät 3.2 ist in einem hinteren Bereich der Arbeitsplatte 3 angeordnet, um auch bei grösseren Medikamentenschachteln ein einwandfreies Einlesen des maschinenlesbaren Medikamenten- und/oder Abgabebehältercodes zu ermöglichen.

Optional kann ein Handscanner, welcher in den Figuren nicht dargestellt ist, vorgesehen sein, um die Flexibilität beim Einlesen zusätzlich zu erhöhen. Der Handscanner kann in einem entsprechenden Fach der Arbeitsplatte 3, an der Seitenwand des Schranks 2 oder auf einem Lagerregal 2.1 angeordnet sein und eine drahtlose Schnittstelle aufweisen, insbesondere zur Kommunikation mit der elektronischen Schaltung 2.5. Das Lesegerät 3.2 und/oder der Handscanner sind eingerichtet, um maschinenlesbare Medikamenten-und/oder Abgabebehältercodes, welche an einem Medikamentenabgabebehälter 4 oder an Medikamentenschachteln angebracht sind, zu erfassen. Es können auch maschinenlesbare Patientencodes von Patienten-Karten oder maschinenlesbare Personalcodes von Personalausweisen der Pflegepersonen erfasst werden.

An der Arbeitsplatte 3 kann ferner eine Einfüllkontrolleinheit 3.5 angebracht sein, um das Einfüllen von Medikamenten in den Medikamentenabgabebehälter zu überwachen. Die Einfüllkontrolleinheit kann an einem hinteren Teil der Arbeitsplatte 3 angeordnet sein. Alternativ ist die Einfüllkontrolleinheit 3.4 bei der Halterung 3.3 oder an einem oberen Teil des Schranks 2 angebracht.

Nicht gezeigt in Fig. 1 ist ein Drucker oder ein anderes Kennzeichnungsgerät, um Abgabebehältercodes wie z.B. 1D- oder 2D-Barcodes insbesondere auf Haftetiketten zu drucken oder RFID-Etiketten zu programmieren, um diese z.B. an einem Medikamentenabgabebehälter 4 anzubringen.

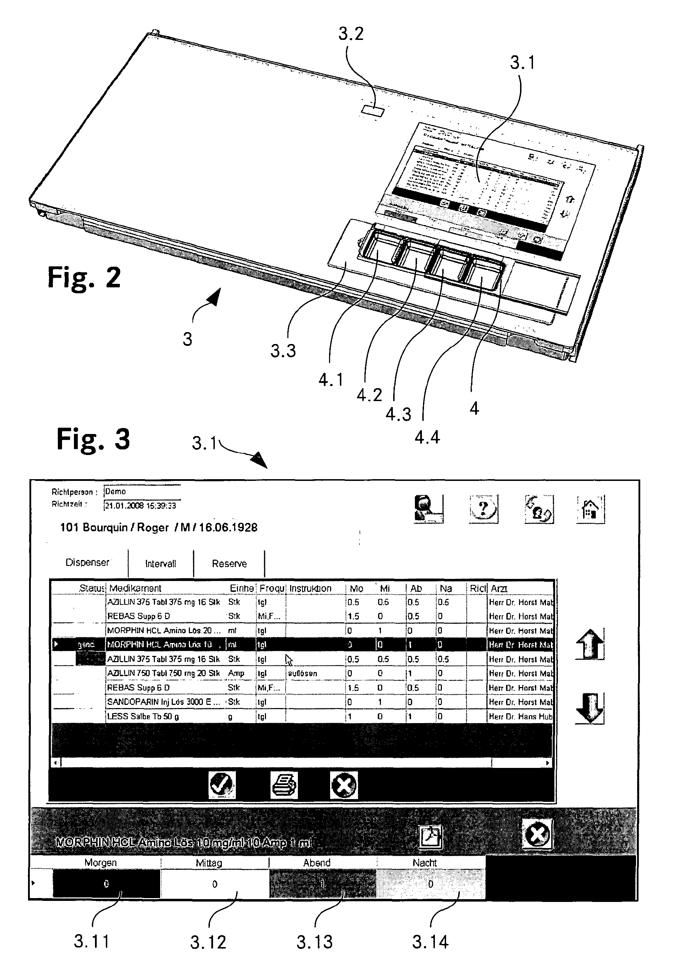

Fig. 2 zeigt eine perspektivische Ansicht einer Arbeitsplatte 3, welche einen eingebauten Bildschirm 3.1, ein Lesegerät 3.2 und einen Medikamentenbehälter 4, welcher in einer Halterung 3.3 neben dem Bildschirm 3.1 angeordnet ist, aufweist. Die Arbeitsplatte 3 ist auf der gesamten Oberfläche mit einer Glasplatte abgedeckt, welche bei der Halterung 3.3 einen Ausschnitt aufweist. Als Glas kann ein bruchsicheres, entspiegeltes, extraweisses Glass der Dicke 4 mm verwendet werden, welches im Bereich des Bildschirms 3.1 und des Lesegeräts 3.2 farblos ist. Die Halterung 3.3 ist austauschbar, sodass die Arbeitsplatte 3 einfach an Medikamentenabgabebehälter 4 mit unterschiedlichen Dimensionen angepasst werden kann. Dabei ist die Halterung 3.3 derart ausgeführt, dass eine Oberkante des Medikamentenabgabebehälters 4 an den Bildschirm 3.1 anschliesst. Wie in Fig. 2 durch unterschiedliche Grautöne angedeutet, können die Fächer 4.1, 4.2, 4.3, 4.4 des Medikamentenabgabebehälters unterschiedliche Farben aufweisen, also z.B. blau, gelb, rot und grün, welche wie nachfolgend beschrieben entsprechenden auf dem Bildschirm 3.1 dargestellten Flächen entsprechen.

Der Schrank 2 kann mehrere Schrankelemente (bzw. Schrankmodule) aufweisen, um auch für einen grossen Medikamentenvorrat genügend Platz zu bieten, wobei die Schrankelemente standardisierte Höhen, Breiten und Tiefen haben. Die ausziehbare Arbeitsplatte 3 ist dabei an die Dimensionen eines Schrankelements angepasst. Die Breite der Arbeitsplatte entspricht im Wesentlichen dem Innenmass der Breite des Schranks. Der Schrank ist tiefer als die Arbeitsplatte.

Fig. 3 zeigt eine Ansicht des Bildschirms 3.1. Wie aus Fig. 2 ersichtlich, grenzt der Medikamentenabgabebehälter 4 direkt an die Unterkante des in Fig. 3 gezeigten Bildschirms 3.1, wobei am unteren Rand des Bildschirms 3.1 Felder 3.11, 3.12, 3.13, 3.14 in unterschiedlichen Farben oder in heller Farbe angezeigt sind, welche den Farben der Fächer 4.1, 4.2, 4.3, 4.4 des Medikamentenabgabebehälters 4 entsprechen. Auf dem berührungsempfindlichen Bildschirm werden Informationen und Bedienelemente angezeigt zur Durchführung der nachfolgend dargestellten Prozeduren. Die Steuerung des Bildschirms erfolgt durch die elektronische Schaltung 2.5, welche dafür geeignete Elektronikschaltungen umfasst. In einer bevorzugten Ausführungsform ist die elektronische Schaltung 2.5 als handelsüblicher Industrie-PC (Personal Computer) aufgebaut, welcher Mittel umfasst, um ein Betriebssystem (wie beispielsweise ein Windows XP®-, Vista®-, Windows7®-Betriebssystem oder auch entsprechende Embeded Systeme wie Windows XP® Embedded) abzuarbeiten sowie ablauffähige Softwaremodule (wie z.B. in Framework 3 oder Visual Studio 2008® entwickelte Softwaremodule). Der PC weist verschiedene Anschlüsse auf, welche in einer Schiene in der Rückwand des Schranks 2 bis zur Arbeitsfläche 3 geführt sein können. So kann der PC insbesondere Anschlüsse für Ethernet, Wireless, Power, RS232C (für den Anschluss eines Handlesegeräts), Centronics (für den Anschluss eines Druckers), Bluetooth und USB aufweisen. Solche Anschlüsse (z.B. USB) können auch mehrfach ausgeführt sein, um mehrere Peripheriegeräte zu bedienen.

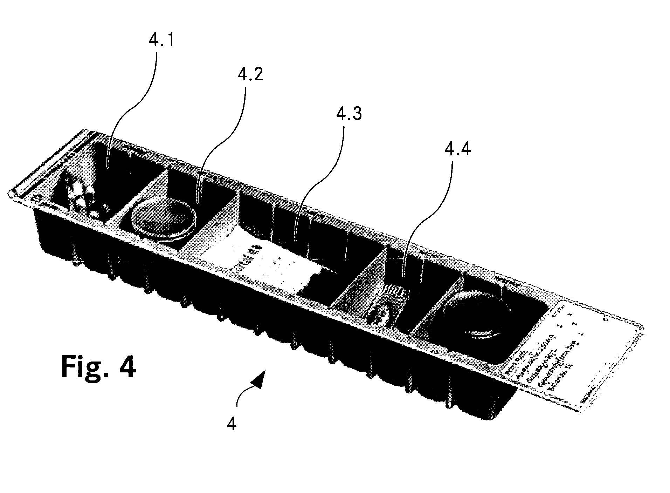

Fig. 4 zeigt eine perspektivische Ansicht eines Medikamentenabgabebehälters 4 mit den vier Fächern 4.1, 4.2, 4.3, 4.4 sowie einem fünften Fach, welche zur Aufnahme von Medikamenten eingerichtet sind. Im Fachjargon wird ein solcher Medikamentenabgabebehälter 4 auch als Dispenser bezeichnet. Die Medikamente können insbesondere als Pillen zur Verfügung stehen. In entsprechenden Dosen oder Ampullen können aber auch flüssige Medikamente im Medikamentenabgabebehälter 4 angeordnet sein. Statt Medikamenten können auch andere für die Versorgung eines Patienten notwendige medizinische Hilfsmittel wie beispielsweise Pflaster, Reinigungswatte, Scheren, Spritzen, etc. im Medikamentenabgabebehälter 4 angeordnet sein. Wie in Fig. 4 skizziert, kann die Facheinteilung des Medikamentenabgabebehälters 4 verändert werden, sodass grössere respektive kleinere Fächer für grössere respektive kleinere Medikamentenmengen oder medizinische Hilfsmittel vorgesehen sind. Der Medikamentenabgabebehälter 4 ist mittels eines Schiebedeckels gasdicht und spritzwasserdicht verschliessbar. Der Schiebedeckel ist dabei nur in einer Richtung bedienbar, sodass die Fächer 4.1, 4.2, 4.3, 4.4 in der Reihenfolge nacheinander geöffnet werden. Die Fächer 4.1, 4.2, 4.3 können beispielsweise entsprechend der Ausgabezeiten "Morgen", "Mittag" und "Abend" befüllt sein und das Fach 4.4 oder weitere Fächer können für eine Reserve oder Nachtabgabe vorgesehen sein.

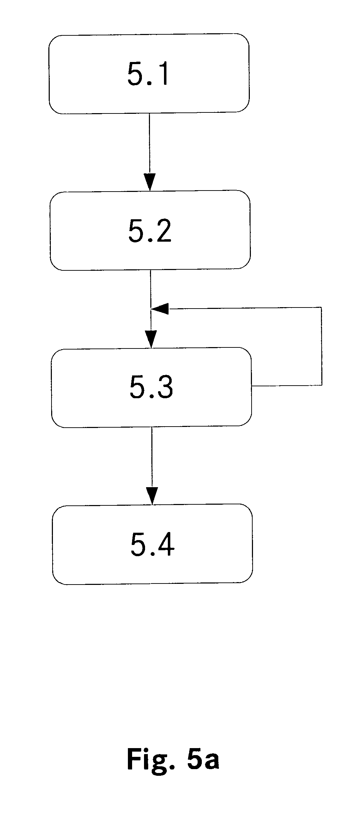

Fig. 5a zeigt schematisch ein Flussdiagramm einer Software zum Steuern des Richtens von Medikamenten.

Im Schritt 5.1 startet die Software, nachdem die Pflegeperson den Schrank 2 geöffnet und die Arbeitsplatte 3 herausgezogen hat. Dies erfolgt durch die automatische oder manuelle Inbetriebnahme der elektronischen Schaltung 2.5, also insbesondere des PCs. Die Pflegeperson wird durch eine Anzeige auf dem Bildschirm 3.1 aufgefordert, sich zu identifizieren, beispielsweise indem ein maschinenlesbarer Personalcode wie beispielsweise ein RFID Personalcode vom Lesegerät 3.2 erfasst oder ein Fingerabdruck eingelesen wird.

Im Schritt 5.2 selektiert die Software einen Patienten, für welchen Medikamente gerichtet werden sollen. Diese Selektion kann automatisch durch die Software nach bestimmten Kriterien wie z.B. dem nächsten Termin für eine Medikamentenabgabe oder durch das Scannen des Abgabebehältercodes und/oder letzten Richtcodes erfolgen. Alternativ wird auf dem Bildschirm 3.1 eine Auswahl von Patienten angezeigt und die Pflegeperson selektiert einen Patienten durch eine entsprechende Eingabe am berührungsempfindlichen Bildschirm 3.1. In einer anderen Variante wird auf die Pflegeperson durch eine entsprechende Anzeige auf dem Bildschirm 3.1 aufgefordert einen Medikamentenabgabebehälter 4 auszuwählen und den daran angebrachten Abgabebehältercode mit maschinenlesbarem Patientencode mit dem Lesegerät 3.2 einzulesen. Anschliessend wird auf dem Bildschirm angezeigt, entweder einen neuen oder den ausgewählten Medikamentenabgabebehälter in die Halterung 3.3 der Arbeitsplatte 3 einzulegen.

Im Schritt 5.3 wird auf dem Bildschirm ein Medikament angezeigt, welches aus dem Medikamentenvorrat des Schranks entnommen werden soll. Die Pflegeperson wird durch eine entsprechende Anzeige auf dem Bildschirm aufgefordert, den an der Medikamentenschachtel angebrachten maschinenlesbaren Medikamentencode mit dem Lesegerät 3.2 einzulesen. Die Software registriert, ob der korrekte Medikamentencode eingelesen wurde. Ist dies nicht der Fall, dann wird durch die Software ein Alarm ausgelöst, indem auf dem Bildschirm eine entsprechende Meldung angezeigt wird und/oder indem ein akustisches Signal ausgelöst wird. Bei einem korrekten Medikamentencode wird durch die Software auf dem Bildschirm angezeigt, in welches der Fächer des Medikamentenabgabebehälters 4 das Medikament zu legen ist. Statt der Anzeige mit auf dem Bildschirm kann mit einem Beleuchtungsgerät wie einem Lasergerät das Fach beleuchtet und angezeigt werden. Zusätzlich kann eine Menge von Einheiten angezeigt werden, welche in das Fach zu legen ist. Durch eine Anzeige einer Aufforderung zum Quittieren oder durch eine Einfüllkontrolleinheit 3.5 wird festgestellt, ob alle Medikamenteneinheiten in die entsprechenden Fächer gelegt wurden. Darauf wird am Bildschirm das nächste zu richtende Medikament angezeigt. Der Schritt 5.3 wiederholt sich so lange, bis alle Medikamente gerichtet sind.

Im Schritt 5.4 steuert die Software gegebenenfalls einen Drucker an und es wird ein Barcode oder RFID Etikette mit einem Abgabebehältercode ausgedruckt oder hergestellt und vorzugsweise zur Dokumentation mit der Kamera ein Bild des gerichteten Abgabebehälters gemacht, das elektronisch aufbewahrt wird. Durch eine entsprechende Anzeige am Bildschirm 3.2 wird die Pflegeperson aufgefordert, die Etikette am Medikamentenabgabebehälter 4 anzubringen, diesen mit einem Deckel zu verschliessen und auf ein Tablar mit gerichteten Medikamentenabgabebehältern zu stellen.

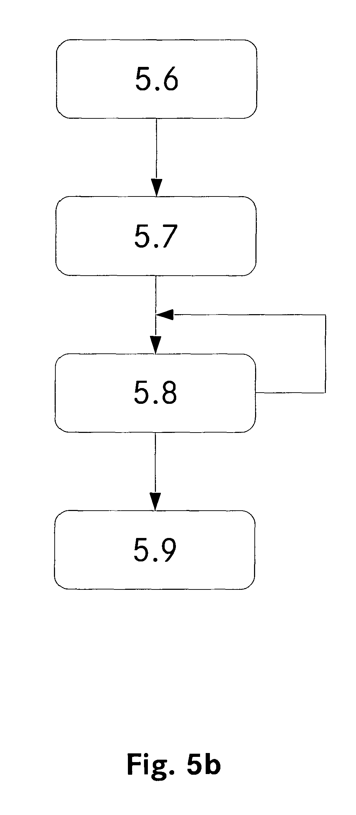

Fig. 5b zeigt schematisch ein Flussdiagramm einer Software zum Steuern des Verteilens von Medikamenten. Ausgangspunkt des Verteilens ist, dass die Medikamentenabgabebehälter 4 in einer mobilen Verteileinrichtung, also insbesondere in einem Wagen oder in einer tragbaren Einrichtung mit Schubladen oder Tablaren, angeordnet sind. Der Wagen umfasst einen Laptop mit einem Touchscreen oder ein anderes mobiles Gerät, auf welchem die Software zum Steuern des Verteilens von Medikamenten abläuft, sowie ein Lesegerät zum Einlesen von maschinenlesbaren Medikamenten-, Patienten- und/oder Personalcodes.

Im Schritt 5.6 fordert die Software eine Pflegeperson auf, sich zu identifizieren, beispielsweise wiederum durch das Einlesen eines maschinenlesbaren Personalcodes oder eines Fingerabdrucks.

Im Schritt 5.7 wird durch die Software ein Patient ausgewählt, für welchen Medikamente verteilt werden sollen. Dies kann automatisch durch die Software erfolgen, indem ein nächster Patient aufgrund einer Bettnummer oder einer Zeitangabe ausgewählt wird. Alternativ kann ein Patient ausgewählt werden, indem die Software die Pflegeperson auffordert einen Patienten auszuwählen, beispielsweise durch eine entsprechende Eingabe mit Eingabemittel des Laptops oder durch das Einlesen eines maschinenlesbaren Patientencodes einer am Bett angebrachten Patientenkennzeichnung. Durch die Software wird anschliessend auf dem Bildschirm angezeigt, welcher Medikamentenabgabebehälter aus den Schubladen oder Tablaren des Wagens entnommen werden muss. Die Pflegeperson wird durch eine entsprechende Anzeige aufgefordert, den am Medikamentenabgabebehälter angebrachten maschinenlesbaren Abgabebehältercode und/oder Richtcode mit dem Lesegerät einzulesen. Die Software registriert, ob der korrekte Medikamentenabgabebehälter entnommen wurde. Falls dies nicht zutrifft, löst die Software gegebenenfalls einen Alarm aus (Anzeige am Bildschirm, akustisches Signal).

Im Schritt 5.8 fordert die Software die Pflegeperson mit einer entsprechenden Anzeige am Bildschirm auf, Medikamente aus dem Medikamentenabgabebehälter zu entnehmen und dem Patienten zu übergeben oder zu verabreichen. Dies kann in einem Schritt erfolgen, indem die Software die Pflegeperson auffordert, alle Medikamente aus einem Fach des Medikamentenabgabebehälters auszublistern und in einen Becher umzufüllen und den Becher dem Patienten zu übergeben. Auch der Becher kann mit einem Barcode-Label versehen sein. Die Software kann die Pflegeperson auch auffordern, die Medikamente einzeln zu übergeben oder zu verabreichen, dies insbesondere dann, wenn die Verabreichung eines Medikaments mit zusätzlichen Handlungen wie dem Einstechen einer Spritze oder dem Aufbrechen einer Ampulle verbunden ist. In diesem Fall kann die Software die einzelnen Schritte auf dem Bildschirm anzeigen und die Pflegeperson auffordern, eine Handlung jeweils zu quittieren. Der Schritt 5.8 wird von der Software solange wiederholt, bis alle zu einem bestimmten Zeitpunkt zu verabreichenden Medikamente übergeben oder verabreicht sind.

Im Schritt 5.9 fordert die Software die Pflegeperson durch eine entsprechende Anzeige am Bildschirm auf, den Medikamentenabgabebehälter gegebenenfalls in den Wagen zurückzulegen und am Patienten zu überprüfen, dass durch die Verabreichung der Medikamente keine Nebenwirkungen entstanden sind. Dabei können von der Software spezifische Angaben wie der Überwachung des Puls, der Körpertemperatur oder der Atemfrequenz gemacht werden, welche von der Pflegeperson zu überprüfen. Diese Angaben können von einer Pflegesoftware entnommen werden. Die Software kann durch eine Anzeige auf dem Bildschirm die Pflegeperson auffordern, die Überwachung von Nebenwirkungen zu quittieren, beispielsweise indem eine Pulsfrequenz, eine Körpertemperatur oder eine Atemfrequenz eingegeben werden muss.

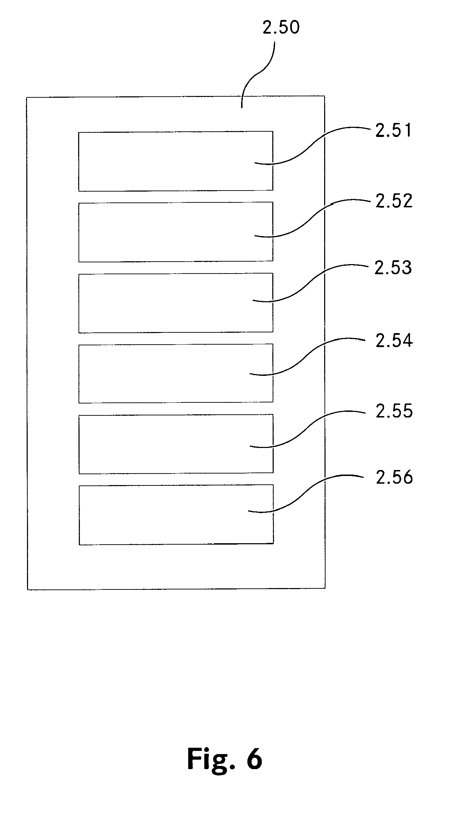

Fig. 6 zeigt schematisch ein Computerprogrammprodukt 2.50 umfassend eines oder mehrere Softwaremodule. Diese umfassen das Steuermodul 2.51, das Codeerfassungsmodul 2.52, das Einfüllkontrollmodul 2.53, das Selektionsmodul 2.54, das Anordnungsmodul 2.55 und/oder das Vergleichsmodul 2.56, deren Aufgaben und Funktionen an anderer Stelle detailliert beschrieben sind. Das Computerprogrammprodukt 2.50 ist insbesondere als ausführbares Programm auf einer Speichereinrichtung wie z.B. einer Harddisk der elektronischen Schaltung 2.5 abgespeichert.

Beim Richten von Medikamenten identifiziert sich die Pflegeperson zunächst gegenüber der Medikamentenrichtstation 1. Dies kann wie im Schritt 5.1 beschrieben durch die Eingabe eines Benutzernamens und Passworts oder durch das Einlesen eines maschinenlesbaren Personalcodes, welcher der Pflegeperson zugeordnet ist, erfolgen. Der maschinenlesbare Personalcode kann durch ein elektronisches Schloss 2.3 des Schranks 2, durch das Lesegerät 3.2 oder durch irgendeine andere Vorrichtung erfasst werden. In einer optimalen Weise wird die Anmeldung am elektronischen Schloss mit dem Personalcode auch gleich für die Anmeldung am PC respektive der elektronischen Schaltung 2.5 verwendet. Danach wählt die Pflegeperson die Abgabezeiten, für welche das Richten von Medikamenten durchgeführt werden soll, d.h. beispielsweise für den Morgen, Mittag und Abend des kommenden Tags.

Dann wählt die Pflegeperson einen Medikamentenabgabebehälter 4. Falls es sich um einen bestehenden Patienten handelt, werden dessen Patientendaten aufgerufen, beispielsweise durch das Einlesen mit dem Lesegerät 3.2 eines am Medikamentenabgabebehälter 4 angebrachten Abgabebehältercodes und/oder letzten Richtcodes, welcher dem Patienten zugeordnet ist. Alternativ werden Patientendaten eines Patienten wie im Schritt 5.2 beschrieben durch eine entsprechende Eingabe am Bildschirm 3.1 aufgerufen. Durch eine entsprechende Datenbankabfrage werden die Medikamente ermittelt, welche für die gewählten Abgabezeiten zu Richten sind.

Je nach der Menge oder der Grösse der Medikamente kann am Bildschirm 3.1 ein Vorschlag dargestellt werden, um die Einteilung der Fächer 4.1, 4.2, 4.3, 4.4 des Medikamentenabgabebehälters 4 optimal einzustellen. Alternativ kann durch die Pflegeperson eine Einteilung der Fächer 4.1, 4.2, 4.3, 4.4 gewählt werden und durch eine entsprechende Eingabe am Bildschirm 3.1 kann die gewählte Einteilung der Fächer 4.1, 4.2, 4.3, 4.4 eingegeben werden.

Nun werden am Bildschirm 3.1 die medizinischen Verordnungen angezeigt. Die Pflegeperson entnimmt wie im Schritt 5.3 beschrieben das erste verschriebene Medikament aus dem Schrank 2 und durch das Einlesen eines maschinenlesbaren Medikamentencodes wird überprüft, ob es sich um das richtige Medikament handelt. Das Medikament kann in einer Originalpackung, in einem Blister oder in einer Unit-Dose Verpackung vorliegen. Stimmt das Medikament mit der Verordnung überein, werden am Bildschirm die Anzahl und das oder die Fächer 4.1, 4.2, 4.3, 4.4 des Medikamentenabgabebehälters 4 angezeigt. Dabei kann die Anzeige derart erfolgen, dass am Bildschirm 3.1 nur ein Bereich direkt beim entsprechenden Fach 4.1, 4.2, 4.3, 4.4 aufleuchtet oder blinkt, zusammen mit einer Anzahl oder einem Bild der in dieses Fach zu legende Medikamente. Stattdessen kann mit einer Beleuchtungseinheit (wie z.B. einem Laserpointer) das Fach beleuchtet werden, in welches das Medikament zu legen ist. Die Pflegeperson legt die Medikamente in die entsprechenden Fächer 4.1, 4.2, 4.3, 4.4 und bestätigt dies gegebenenfalls durch eine entsprechend Eingabe am Bildschirm 3.1.

Nachdem das Richten von Medikamenten für einen Medikamentenabgabebehälter 4 abgeschlossen ist, wird dieser wie im Schritt 5.4 beschrieben mit einem Klarsicht-Schiebedeckel verschlossen und es wird gegebenenfalls eine Etikette mit einem Abgabebehältercode und/oder einem Richtcode und weiteren Angaben wie dem Patientennamen ausgedruckt und am Medikamentenabgabebehälter 4 angebracht. Die gerichteten Medikamente oder Materialien werden zur Leistungserfassung in einer Datenbank abgespeichert.

Die Abgabe von Medikamenten erfolgt mit dem Medikamentenabgabebehälter 4 am Patientenbett. Auf einem Wagen sind ein Laptop mit Touchscreen und Lesegerät für maschinenlesbare Personal-, Abgabebehältercodes und/oder Richtcodes angeordnet. Stattdessen kann auch ein Handheld-Computer und "WIEGAND" Medikamentenverteiltablett vorgesehen sein. Die Pflegeperson identifiziert sich wie im Schritt 5.6 beschrieben durch die Eingabe eines Benutzernamens und Passworts, durch die Erfassung eines biometrischen Merkmals wie eines Fingerabdrucks oder durch die Erfassung eines maschinenlesbaren Personalcodes, welcher der Pflegeperson eindeutig zugeordnet ist. Dann wird der Abgabebehältercode und/oder Richtcode mit dem maschinenlesbaren Patientencode des Medikamentenabgabebehälters 4 eingelesen und entsprechend der Tageszeit wird das Fach 4.1, 4.2, 4.3, 4.4 angezeigt, welches die aktuell zu verabreichenden Medikamente enthält. Diese Medikamente werden am Bildschirm des Laptops aufgelistet und angezeigt.

Mit dem Lesegerät wird wie im Schritt 5.7 beschrieben ein Patientencode eingelesen, also beispielsweise ein Barcode, welcher am Bett des Patienten angebracht ist. Bei falschem Patienten erscheint eine optische und/oder akustische Meldung. Die Medikamente werden aus dem entsprechenden Fach des Medikamentenabgabebehälters 4 in einen Becher umgefüllt und dem Patienten zur Einnahme übergeben. Zugleich können am Bildschirm zusätzliche Informationen dargestellt werden, auf welche die Pflegeperson achten muss, nachdem der Patient die Medikamente eingenommen hat. Solche Angaben können beispielsweise die Körpertemperatur, den Puls oder die Atemfrequenz betreffen.

Insbesondere für die Herstellung der erwähnten Abgabebehältercodes und Richtcodes kann ein Drucker zum Drucken von Etiketten beispielsweise auf einem der unteren Lagertablare 2.1 des Schranks 2 angeordnet sein, sodass die gedruckten Etiketten bequem entnommen werden können.

An einem oberen Teil des Schranks 2 kann eine Fotokamera angeordnet sein. Sobald die Pflegeperson das Richten von Medikamenten in einem Medikamentenabgabebehälter 4 abgeschlossen hat, kann durch die Erfassung eines entsprechenden Bildes der Richtprozess dokumentiert werden.

Entsprechend der verwendeten Medikamente kann der Vorrat von Medikamenten, welche im Schrank 2 verfügbar sind, überwacht werden und eine Nachbestellung von Medikamenten kann beispielsweise elektronisch durch die Übermittlung einer entsprechenden Bestellung an eine Apotheke frühzeitig ausgelöst werden.

Zusammenfassend ist festzustellen, dass durch die Erfindung ein System zum Richten und Verteilen von Medikamenten geschaffen wird, welche das Richten von Medikamenten wesentlich vereinfacht und damit die Fehlerquote beim Richten und Verteilen von Medikamenten erheblich vermindert.

## Patentansprüche

1. Vorrichtung zur Unterstützung eines manuellen Richtens von Medikamenten, umfassend:
a) eine Halterung (3.3) für einen Medikamentenabgabebehälter (4), welcher wahlweise in ein oder mehrere Fächer (4.1, 4.2, 4.3, 4.4) unterteilbar ist,
b) ein elektronisch ansteuerbares Fachzeigemittel (3.1, 3.11) zum visuellen Hinweisen auf das zu befüllende Fach (4.1, 4.2, 4.3, 4.4) des Medikamentenabgabebehälters (4), und
c) ein Steuermodul (2.51) zur Steuerung des Fachzeigemittels (3.1, 3.11) aufgrund von vorgegebenen Verschreibungsdaten.

2. Vorrichtung nach Anspruch 1, **dadurch gekennzeichnet, dass** das Fachzeigemittel einen Bildschirm (3.1) umfasst, welcher benachbart zur Halterung (3.3) für den Medikamentenabgabebehälter (4) angebracht ist, um mit mindestens einem der Halterung (3) benachbarten Bildschirmbereich auf mindestens ein Fach (4.1, 4.2, 4.3, 4.4) des Medikamentenabgabebehälters (4) hinzuweisen.

3. Vorrichtung nach Anspruch 1, **dadurch gekennzeichnet, dass** das Fachzeigemittel ein Beleuchtungsmittel (3.4) umfasst, um mindestens ein Fach (4.1, 4.2, 4.3, 4.4) des Medikamentenabgabebehälters (4) zu beleuchten und damit darauf hinzuweisen.

4. Vorrichtung nach einem der Ansprüche 1 bis 3, weiter umfassend ein Lesegerät (3.2) zum Erfassen von maschinenlesbaren Codes, wobei das Lesegerät (3.2) eingerichtet ist, um erfasste Codes an ein Codeerfassungsmodul (2.52) zur Auswertung zu übermitteln.

5. Vorrichtung nach einem der Ansprüche 1 bis 4, weiter umfassend eine Einfüllkontrolleinheit (3.5), welche insbesondere eine Lichtschranke, eine Foto-/Videokamera und/oder ein Distanzmessgerät aufweist, um das Einfüllen eines Medikaments in ein Fach (4.1, 4.2, 4.3, 4.4) des Medikamentenabgabebehälters (4) zu überwachen, wobei die Einfüllkontrolleinheit (3.5) eine Signalisierungseinrichtung umfasst, welche das Überwachungsresultat an ein Einfüllkontrollmodul (2.53) übermittelt, wobei insbesondere ein Bild des fertig gerichteten Medikamentenabgabebehälters (4) zur Dokumentation übermittelt wird.

6. Vorrichtung nach einem der Ansprüche 1 bis 5, **dadurch gekennzeichnet, dass** der Bildschirm (3.1), das Lesegerät (3.2), die Halterung (3.3) und/oder die Einfüllkontrolleinheit (3.5) in eine in eine Medikamentenrichtstation einbaubare Arbeitsplatte (3) eingebaut und/oder daran angebracht ist/sind, wobei insbesondere eine Glasplatte vorgesehen ist, welche die Arbeitsplatte (3) im Wesentlichen ganzflächig abdeckt.

7. System zum Richten und Verteilen von Medikamenten, umfassend eine Medikamentenrichtstation (1) mit:
a) einer Vorrichtung zur Lagerung eines Medikamentenvorrats, insbesondere einem Schrank (2),
b) einer Vorrichtung zur Unterstützung eines manuellen Richtens von Medikamenten nach einem der Ansprüche 1 bis 5,
c) einer elektronischen Schaltung (2.5) umfassend das Steuermodul (2.51), das Codeerfassungsmodul (2.52) und/oder das Einfüllkontrollmodul (2.53) der Vorrichtung nach einem der Ansprüche 1 bis 5, und
d) einem Eingabegerät zur manuellen Steuerung der elektronischen Schaltung (2.5).

8. System nach Anspruch 7, **dadurch gekennzeichnet, dass** der Medikamentenvorrat mehrere Medikamentenbehälter mit je einem daran angebrachten maschinenlesbaren Medikamentencode zur Identifikation eines Medikaments umfasst.

9. System nach einem der Ansprüche 7 bis 8, weiter umfassend eine Mehrzahl von wahlweise in ein oder mehrere Fächer (4.1, 4.2, 4.3, 4.4) unterteilbare Medikamentenabgabebehälter (4) mit daran angebrachtem maschinenlesbaren Abgabebehältercode und/oder Richtcode.

10. System nach einem der Ansprüche 7 bis 9, **dadurch gekennzeichnet, dass** das Eingabegerät einen berührungsempfindlichen Bildschirm (3.1) umfasst.

11. System nach einem der Ansprüche 7 bis 10, weiter umfassend eine Kennzeichnungseinheit, um einen maschinenlesbaren Abgabebehältercode oder Richtcode zu erzeugen und/oder einen maschinenlesbaren Abgabebehältercode oder Richtcode mindestens teilweise zu Verschreibungsdaten zuzuordnen.

12. System nach einem der Ansprüche 7 bis 11, weiter umfassend eine mobile Verteileinrichtung zur Aufnahme und zum Transport von Medikamentenabgabebehältern (4), wobei die mobile Verteileinrichtung eine Anzeigeeinheit, eine Eingabeeinheit und ein Lesegerät zur Erfassung von maschinenlesbaren Codes aufweist.

13. System nach einem der Ansprüche 7 bis 12, weiter umfassend eine Mehrzahl von Patientenkennzeichnungen mit maschinenlesbarem Patientencode.

14. Verfahren zum Betreiben eines Systems nach einem der Ansprüche 7 bis 13, **gekennzeichnet durch** folgende Schritte:
a) elektronische Aufforderung, ein Medikament in den Medikamentenabgabebehälter (4) zu legen, und
b) Anzeige in welches der Fächer (4.1, 4.2, 4.3, 4.4) des Medikamentenabgabebehälters (4) das Medikament zu füllen ist, indem mit den Fachzeigemittel (3.1, 3.11) auf dieses Fach hingewiesen wird.

15. Computerprogrammprodukt (2.50) zur Steuerung eines Systems zum Richten und Verteilen von Medikamenten nach einem der Ansprüche 7 bis 13, umfassend eine oder mehrere der folgenden Softwaremodule:
a) Selektionsmodul (2.54) zum automatischen oder manuellen Selektieren von Verschreibungsdaten aus einer Datenbank mit Verschreibungsdaten,
b) Anordnungsmodul (2.55) zur Aufforderung/Überwachung des Anordnens eines wahlweise in ein oder mehrere Fächer (4.1, 4.2, 4.3, 4.4) unterteilbaren Medikamentenabgabebehälters (4) in der Halterung (3.3) der Vorrichtung zur Unterstützung eines manuellen Richtens von Medikamenten,
c) Codeerfassungsmodul (2.52) zum Einlesen und Erfassen von Codes mit einem Lesegerät (3.2), wie insbesondere maschinenlesbare Medikamentencodes, Abgabebehältercodes, Patientencodes und/oder Personalcodes,
d) Vergleichsmodul (2.56) zum Vergleichen von eingelesenen Codes mit Verschreibungsdaten,
e) Steuermodul (2.51) zum Hinweisen auf ein Fach (4.1, 4.2, 4.3, 4.4) des Medikamentenabgabebehälters (4) mit dem Fachzeigemittel (3.1, 3.11), und
f) Einfüllkontrollmodul (2.53) zur Überwachung des Einfüllens von Medikamenten in ein Fach (4.1, 4.2, 4.3, 4.4) des Medikamentenabgabebehälters (4) mit einer Einfüllkontrolleinheit (3.5).
